# EUROPEAN PATENT SPECIFICATION

(11) **EP 0 668 782 B1**
(45) Date of publication and mention of the grant of the patent: **11.04.2001**
(21) Application number: 93919965.9
(22) Date of filing: 10.08.1993
(51) Int. Cl.: A61K 48/00, C12N 15/00

(54) **COMBINATION OF ANTINEOPLASTIC AGENT AND ANTISENSE OLIGONUCLEOTIDES FOR TREATMENT OF CANCER**
DIE KOMBINATION VON ANTINEOPLASTISCHEN MITTELN UND ANTISENSE-OLIGONUKLEOTIDEN BEI DER BEHANDLUNG VON KREBS
COMBINAISON D'UN AGENT ANTI-NEOPLASIQUE ET D'OLIGONUCLEOTIDES ANTISENS DANS LE TRAITEMENT DU CANCER

(30) Priority: 21.10.1992 US 965671
(43) Date of publication of application: 30.08.1995
(73) Proprietor: TEMPLE UNIVERSITY - OF THE COMMONWEALTH SYSTEM OF HIGHER EDUCATION, Philadelphia, PA 19102 (US); THOMAS JEFFERSON UNIVERSITY, Philadelphia, Pennsylvania 19107-6799 (US)
(72) Inventor: CALABRETTA, Bruno, Philadelphia, PA 19103 (US); SKORSKI, Thomasz, Philadelphia, PA (FR)
(74) Representative: W.P. THOMPSON & CO.
(86) International application number: US9307541
(87) International publication number: WO9408625

(56) References cited:
- WO-A-91/04753
- US-A- 5 055 459
- Science, Volume 253, issued 02 August 1991, C. SZCZYLIK et al., "Selective Inhibition of Leukemia Cell Proliferation by BCR-ABL Antisense Oligodeoxynucleotides", pages 562-565, see page 563.

## Description

### Field of the Invention

The invention relates to therapeutic combinations of anti-cancer pharmaceuticals and antisense oligonucleotides, in particular antisense oligonucleotides which hybridize to mRNA of oncogenes and proto-oncogenes.

### Background of the Invention

Many antineoplastic agents have an unacceptably low therapeutic index, that is, they are unable to inhibit proliferation of cancerous cells at doses which spare significant numbers of normal cells. Therapeutic intervention with such agents runs the risk of debilitating side effects and even death of the patient. There is therefore a need with many antineoplastic agents to be able to reduce the drug dosage without compromising effectiveness.

Bone marrow transplantation comprises the removal of healthy bone marrow cells from a donor and transplantation into a recipient having incomplete, incompetent or diseased bone marrow. The procedure may be used following high-dose chemotherapy and/or radiation treatment to repopulate the patient's bone marrow. Allogenic transplantation comprises transfer of tissue between genetically dissimilar members of the same species. Syngeneic transplantation is the transfer to a genetically identical individual. Autologous transplantation comprises removal and then, following irradiation or chemotherapy, reinfusion of a patient's own bone marrow cells.

Chronic myelogenous leukemia (CML) is a disease of the hematopoietic stem cells, which generates an expanded pool of unipotent progenitors that retain the ability to differentiate during the chronic phase, but undergo severe differentiation arrest during blast crisis. Intensive chemoradiotherapy followed by allogeneic or syngeneic bone marrow transplantation has become effective therapy for patients with chronic myelogenous leukemia (CML) (Thomas et al., Ann. Intern. Med. 104, 155-157 (1986)). If appropriate marrow donors are not available, autologous bone marrow transplantation may be utilized, although the results are not as satisfactory as allogeneic transplantation, perhaps in part due to the presence of contaminating leukemic cells in the reinfused bone marrow autograft (Verafaille et al., Blood 79, 1003-1010 (1992); Degliantoni et al., Blood 65, 753-757 (1985)). Autologous transplantation involves removal and storage of bone marrow from an individual having cancer, and then treating the patient with radiation and/or high dose chemotherapy at a level which results in the destruction of cancer cells, but which also results in the death of normal marrow cells. The stored marrow, which contains pluripotent stem cells capable of regenerating the bone marrow, is then transplanted back into the host to regenerate the bone marrow.

The advantage of coupling chemotherapy with autologous bone marrow rescue is that high doses of drug may be administered. Even with the less than one order of magnitude increase in dose that can be achieved using autologous rescue, the response rates of many tumors are increased. The principal drawback of autologous transplant is the possibility that the marrow used to rescue the patient is contaminated with malignant cells. This is particularly true when the disease condition is itself a malignancy of the bone marrow, or when the disease originates elsewhere in the body and neoplastic cells metastasize to the bone marrow. In CML patients in particular, autologous bone marrow transplantation is associated with a high relapse rate (Butturini and Gale, Br. J. Haematol. 72, 479-485 (1989); Bron and Styckmans, Eur. J. Cancer Clin. Oncol. 25, 163-166 (1989), due most likely to residual clonogenic leukemia cells within the autograft (Apperley et al., Br. J. Haematol. 69, 239-245 (1988)). What is needed is a tumor-specific regimen effective in purging autologous bone marrow grafts of malignant cells prior to return to the donor.

Among candidate chemotherapeutic agents, 4-hydroxyperoxycyclophosphamide (4-HC) and mafosfamide have been shown to be effective purging agents in acute leukemia (Gorin et al., Blood 75, 1606-1614 (1990); Sharkis et al., Blood 55, 5210523 (1980)). Restoration of apparently normal hematopoiesis after treatment of CML marrow cells with 4-HC has been described by one group of investigators (Degliantoni et al., Blood 65, 753-757 (1985)), although another group has questioned the effectiveness of 4-HC or mafosfamide for purging of CML cells from marrows (Mortensen et al., Eur. J. Haematol. 41, 218-222 (1988)).

Molecular strategies are being developed to downregulate unwanted gene expression. One such strategy involves inhibiting gene expression with oligonucleotides complementary in sequence to the messenger RNA of a deleterious target gene. These so-called "antisense" oligonucleotides have been proposed as anti-cancer agents, by targeting various oncogenes or proto-oncogenes. See, for example, U.S. Patent 5,098,890 (c-myb antisense for treating hematologic neoplasms, including use in bone marrow purging); International Patent Application WO 91/03260 (c-abl antisense for treating myeloproliferative disorders); Ratajczak et al., Proc. Natl. Acad. Sci. USA 89, 1710-1714 (1992) (c-kit for inhibiting malignant hematopoietic cell proliferation) ; Szcylick et al., Science 253, 562-565 (1991) (bcr-abl antisense for inhibiting leukemia cell proliferation); Melani et al., Cancer Res. 51, 2897-2901 (1991) (c-myb antisense for inhibiting proliferation of colon cancer cells); and U.S. Patent 5,087,617 which describes bone marrow purging and in vivo therapy using antisense oligonucleotides to a variety of oncogenes and proto-oncogenes. The entire disclosure of each of the aforementioned references is incorporated by reference herein. It has not, however, been suggested to combine an antisense oligonucleotide with a conventional non-oligonucleotide antineoplastic agent for cancer chemotherapy.

### Summary of the Invention

A pharmaceutical composition is provided comprising (A) at least one antisense oligonucleotide which has a nucleotide sequence complementary to at least a portion of the mRNA transcript of an oncogene or proto-oncogene, the oligonucleotide being hybridizable to the mRNA transcript of the oncogene or proto-oncogene, and (B) at least one antineoplastic chemotherapeutic agent other than an antisense oligonucleotide.

The invention also provides the use of a composition comprising
(a) at least one oligonucleotide which has a nucleotide sequence complementary to at least a portion of the mRNA transcript of an oncogene or proto-oncogene, said oligonucleotide being hybridizable to said mRNA transcript; and
(b) at least one antineoplastic chemotherapeutic agent other than an antisense oligonucleotide;
in the manufacture of a medicament for use in the treatment of cancer.

The invention further provides the use of an oligonucleotide which has a nucleotide sequence complementary to at least a portion of the mRNA transcript of an oncogene or proto-oncogene, said oligonucleotide being hybridizable to said mRNA transcript, in the manufacture of a medicament for use, simultaneously or sequentially, with at least one antineoplastic chemotherapeutic agent other than an antisense oligonucleotide, in the treatment of cancer; and the use of an antineoplastic chemotherapeutic agent other than an antisense oligonucleotide in the manufacture of a medicament, for use simultaneously or sequentially, with at least one oligonucleotide which has a nucleotide sequence complementary to at least a portion of the mRNA transcript of an oncogene or proto-oncogene, said oligonucleotide being hybridizable to said mRNA transcript, in the treatment of cancer.

The invention still further provides a method for purging bone marrow of neoplastic cells comprising:
treating bone marrow cells aspirated from an individual afflicted with a neoplastic disease with an effective amount of
(a) at least one oligonucleotide which has a nucleotide sequence complementary to at least a portion of the mRNA transcript of an oncogene or proto-oncogene, said oligonucleotide being hybridizable to said mRNA transcript; and
(b) at least one antineoplastic chemotherapeutic agent other than an antisense oligonucleotide.

According to another embodiment, the invention relates to an in vitro method for inhibiting the proliferation of neoplastic cells characterized by the amplification or expression of a targeted oncogene or proto-oncogene comprising introducing into such cells an artificially-constructed gene which, upon transcription in said cells, produces RNA complementary to at least a portion of the mRNA transcript of the target oncogene or proto-oncogene, and treating such cells with at least one chemotherapeutic agent other than an antisense oligonucleotide. The artificially-constructed gene preferably comprises a transcriptional promotor segment and a segment containing target gene DNA in inverted orientation such that transcription thereof produces the complementary mRNA. The artificially constructed gene may be introduced into the neoplastic cells by, for example, transfection and transduction with a viral vector.

Each antisense oligonucleotide has a nucleotide sequence complementary to at least a portion of the mRNA transcript of the target gene. The oligonucleotide is hybridizable to the mRNA transcript. The oligonucleotide is preferably at least an 8-mer oligonucleotide, that is, an oligomer containing at least 8 nucleotide residues. Except for oligonucleotides generated in situ via transfection with an artificial gene construct, the oligomer preferably contains up to 50 nucleotides. In particular, the oligomer is advantageously a 12-mer to a 40-mer, preferably an oligodeoxynucleotide. While oligonucleotides smaller than 12-mers may be utilized, they are statistically more likely to hybridize with non-targeted sequences, and for this reason may be less specific. In addition, a single mismatch may destabilize the hybrid. While oligonucleotides larger than 40-mers may be utilized, uptake may be more difficult. Moreover, partial matching of long sequences may lead to non-specific hybridization, and non-specific effects. Most preferably, the oligonucleotide is a 15- to 30-mer oligodeoxynucleotide, more advantageously an 18- to 26-mer.

While in principle oligonucleotides having a sequence complementary to any region of the mRNA of the target gene find utility in the present invention, oligonucleotides complementary to a portion of the target gene mRNA transcript including the translation initiation codon are particularly preferred. Also preferred are oligonucleotides complementary to a portion of the target gene mRNA transcript lying within about 50 nucleotides (preferably within about 40 nucleotides) upstream (the 5'→3' direction), or downstream (the 3'→5' direction) from the translation initiation codon.

As used in the herein specification and appended claims, unless otherwise indicated, the term "oligonucleotide" includes both oligomers of ribonucleotides, i.e., oligoribonucleotides, and oligomers of deoxyribonucleotides, i.e., oligodeoxyribonucleotides (also referred to herein as "oligodeoxynucleotides"). Oligodeoxynucleotides are preferred.

As used herein, unless otherwise indicated, the term "oligonucleotide" also includes oligomers which may be large enough to be termed "polynucleotides".

The terms "oligonucleotide" and "oligodeoxynucleotide" include not only oligomers and polymers of the common biologically significant nucleotides, i.e., the nucleotides adenine ("A"), deoxyadenine ("dA"), guanine ("G"), deoxyguanine ("dG"), cytosine ("C"), deoxycytosine ("dC"), thymine ("T") and uracil ("U"), but also include oligomers and polymers hybridizable to the target mRNA transcript which may contain other nucleotides. Likewise, the terms "oligonucleotide" and "oligodeoxynucleotide" includes oligomers and polymers wherein one or more purine or pyrimidine moieties, sugar moieties or internucleotide linkages is chemically modified. The term "oligonucleotide" is thus understood to also include oligomers which may properly be designated as "oligonucleosides" because of modification of the internucleotide phosphodiester bond. Such modified oligonucleotides include, for example, the alkylphosphonate oligonucleosides, discussed below.

The term "phosphorothioate oligonucleotide" means an oligonucleotide wherein one or more of the internucleotide linkages is a phosphorothioate group, 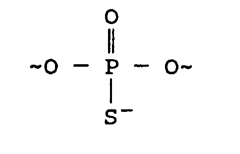 as opposed to the phosphodiester group 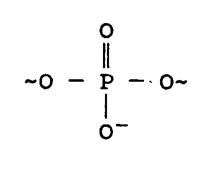 which is characteristic of unmodified oligonucleotides.

By "alkylphosphonate oligonucleoside" is meant an oligonucleotide wherein one or more of the internucleotide linkages is an alkylphosphonate group, 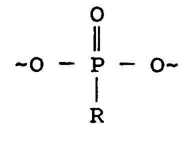 wherein R is an alkyl group, preferably methyl or ethyl.

The term "downstream" when used in reference to a direction along a nucleotide sequence means the 5'→3' direction. Similarly, the term "upstream" means the 3'→5' direction.

The term "target gene mRNA transcript" means the presently known mRNA transcript of the targeted oncogene or proto-oncogene and all variations thereof, or any further transcripts which may be elucidated.

The term "oncogene" means a genetic sequence, i.e. nucleotide sequence, whose expression within a cell provides a function, including one of several functions, in the steps of the transformation leading from a normal cell into a tumor cell.

The term "proto-oncogene" means a genetic sequence residing in the normal genome of a normal, non-tumor cell, which has the potential, when altered in the appropriate manner, of becoming an oncogene.

### Description of the Figures

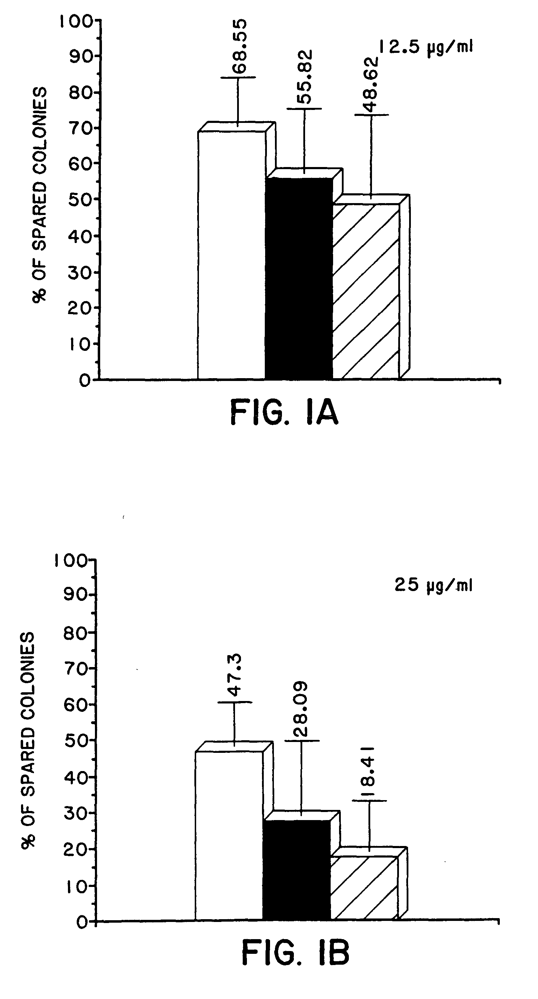

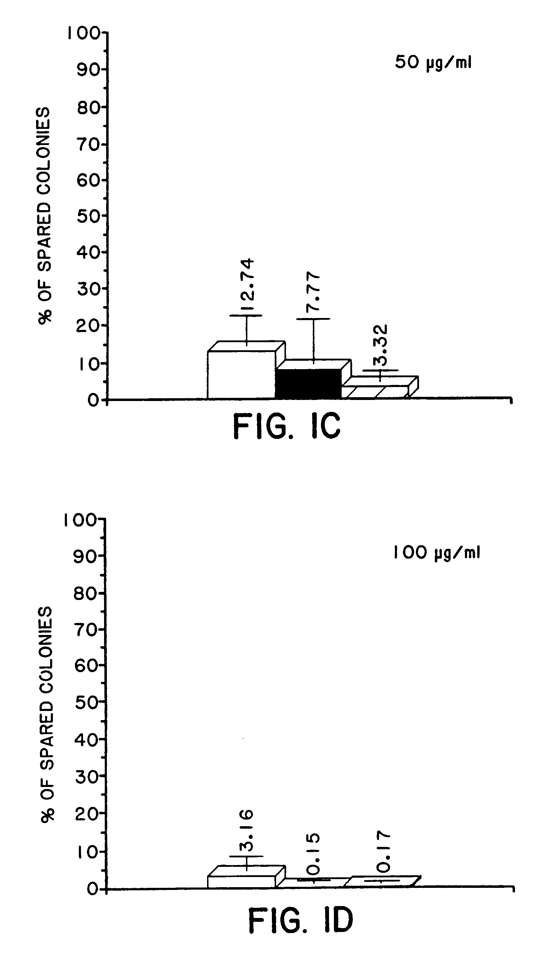

Figs. 1A through 1D comprise the results of colony assays of treatment of the following cell types with mafosfamide: adherent cell-depleted mononuclear cells (A⁻T⁻MNC) from healthy volunteers (hollow); MNC from CML patients in blast crisis (CML-BC) (solid); MNC from patients in chronic phase CML (CML-CP) (crosshatch) . Mafosfamide concentrations: 1A, 12.5 *µ*g/ml; 1B, 25 *µ*g/ml; 1C, 50 *µ*g/ml; and 1D, 100 *µ*g/ml. Bars represent the mean ± standard deviation.

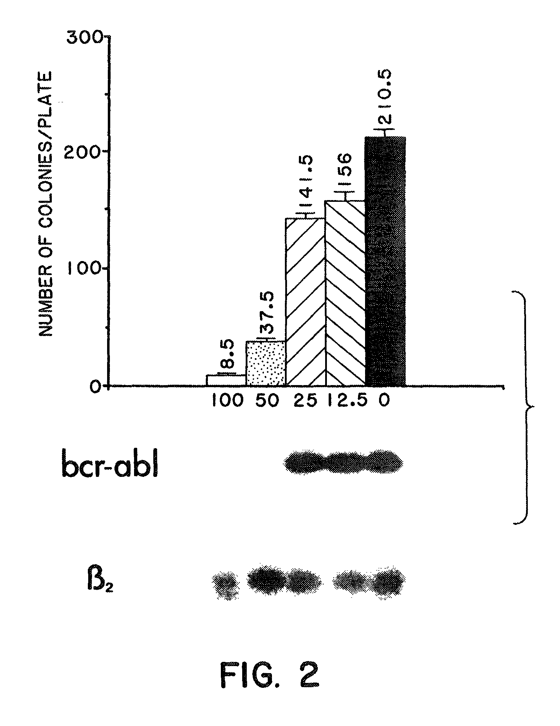

Fig. 2 (graph) represents a colony assay of a 1:1 mixture of normal A⁻T⁻MNC and CML-BC cells incubated with increasing concentrations of mafosfamide (*µ*g/ml). Bars represent the mean ± standard deviation. RT-PCR products were amplified from the same cell mixtures, blotted and probed with hybridization probes for bcr-abl and β₂-microglobulin mRNA (bottom).

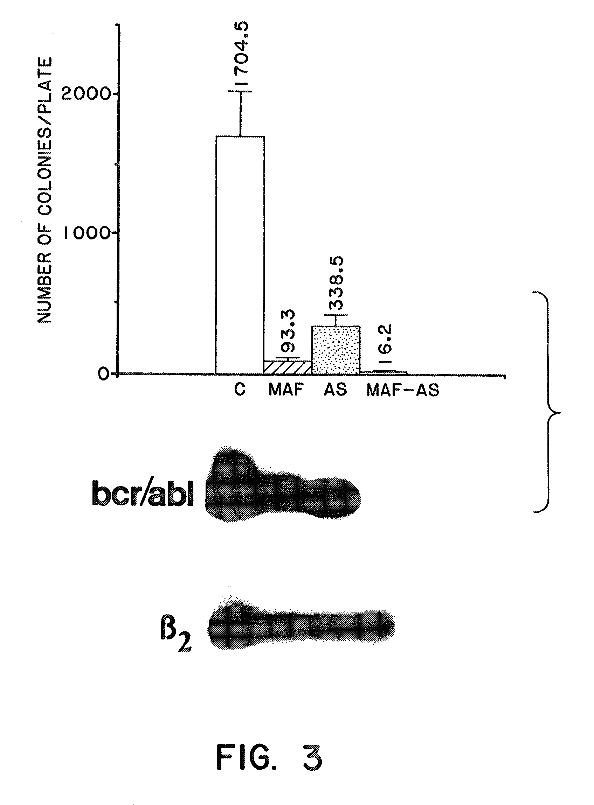

Fig. 3 (graph) represents a colony assay of a 1:1 mixture of normal A⁻T⁻MNC and the Philadelphia chromosome-positive cell line BV173 (genotype b2a2) incubated with 2.5 *µ*g/ml mafosfamide ("MAF"); 80 *µ*g/ml at time zero, followed by 40 *µ*g/ml at 18 hours, and 40 *µ*g/ml at 40 hours, of an antisense oligonucleotide complementary to the breakpoint junction of the b2a2 bcr-abl mRNA transcript (SEQ ID NO:1) ("AS") ; the same concentration of the corresponding sense oligomer (SEQ ID NO:2) as a control ("C"); and the combination of mafosfamide (2.5 *µ*g/ml) and the antisense oligomer (80 *µ*g/ml at time zero, followed by 40 *µ*g/ml at 18 hours and 40 *µ*g/ml at 40 hours) ("MAF+AS"). Bars represent the mean ± standard deviation. RT-PCR products were transcribed from the same cell mixtures, blotted and probed with hybridization probes for bcr-abl and β₂-microglobulin mRNA.

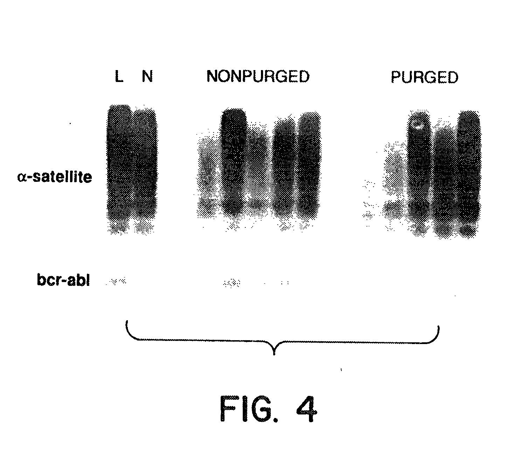

Fig. 4 represents the RT-PCR detection of human α-satellite DNA and bcr-abl mRNA in colonies of marrow cells of sublethally irradiated SCID mice after injection with a 1:1 mixture of normal human A⁻T⁻MNC and BV173 cells untreated ("NONPURGED") or pretreated ("PURGED") with a combination of mafosfamide (2.5 *µ*g/ml) and antisense oligonucleotide (SEQ ID NO:1; 80 *µ* g/ml at time zero, followed by 40 *µ*g/ml at 18 hours and 40 *µ*g/ml at 40 hours). Colonies of BV173 leukemia (L) or normal bone marrow (N) cells served as positive and negative controls for bcr-abl mRNA detection.

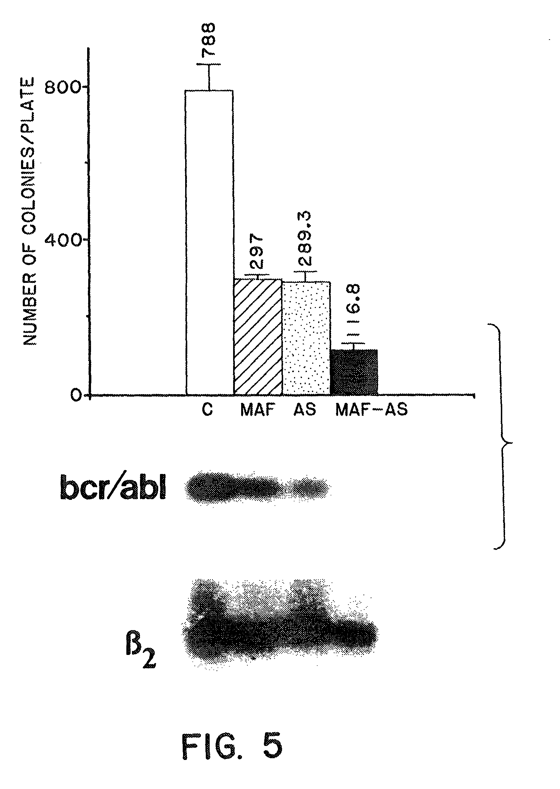

Fig. 5 (graph) represents a colony assay on a 1:1 mixture of normal A⁻T⁻MNC and CML-BC cells (genotype b2a2) incubated with 25 *µ*g/ml mafosfamide ("MAF") ; (80 *µ*g/ml at time zero, followed by 40 *µ*g/ml at 18 hours and 40 *µ*g/ml at 40 hours) of antisense oligonucleotide (SEQ ID NO:1) ("AS"); the same concentration of the corresponding sense oligomer (SEQ ID NO:2) as a control ("C") ; and the combination of mafosfamide (25 *µ*g/ml) and antisense oligomer (80 *µ* g/ml at time zero, followed by 40 *µ*g/ml at 18 hours and 40*µ*g/ml at 40 hours) ("MAF+AS"). Bars represent the mean ± standard deviation. Reverse transcriptase-polymerase chain reaction (RT-PCR) products were transcribed from the same cell mixtures, blotted and probed with hybridization probes for bcr-abl and β₂-microglobulin mRNA.

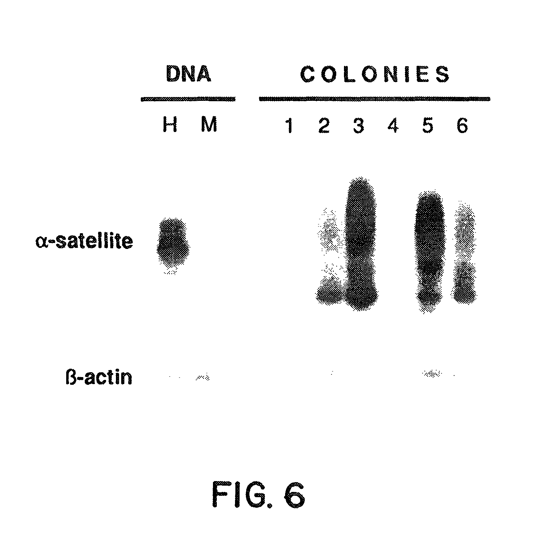

Fig. 6 represents the RT-PCR detection of human α-satellite DNA and β-actin mRNA in six colonies of marrow cells obtained from sublethally irradiated BNX mice after injection with normal human A⁻T⁻MNC pretreated with a combination of mafosfamide (25 *µ*g/ml) and antisense oligonucleotide (SEQ ID NO:1; 80 *µ*g/ml at time zero, followed by 40 *µ*g/ml at 18 hours and 40 *µ*g/ml at 40 hours). DNA from human (H) and murine (M) peripheral blood mononuclear cells served as positive and negative controls, respectively.

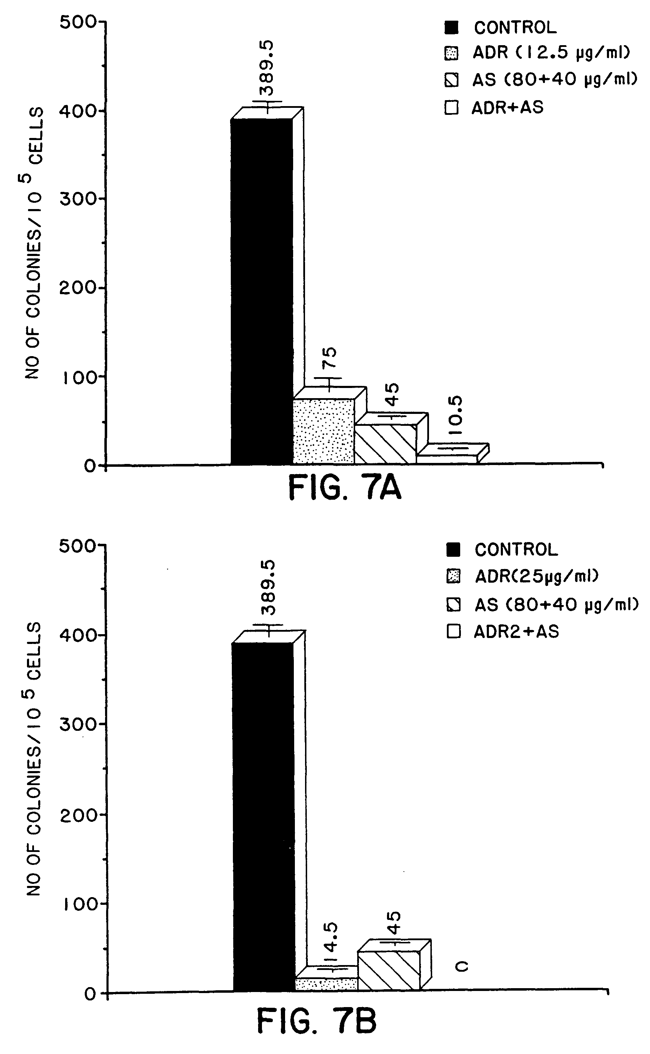

Fig. 7A represents a colony assay of BV173 cells incubated with 12.5 *µ*g/ml adriamycin ("ADR"); 80 *µ*g/ml for 18 hours (followed by 40 *µ*g/ml for the next 24 hours) of antisense oligonucleotide (SEQ ID NO:1) ("AS"); the same concentration of the corresponding sense oligomer (SEQ ID NO:2) as a control ("Control"); and the combination of adriamycin (12.5 *µ*g/ml) and antisense oligomer (80 *µ*g/ml for 18 hours, followed by 40 *µ*g/ml for the next 24 hours) ("ADR+AS"). Fig. 7B is similar to Fig. 7A, except that the concentration of adriamycin is increased to 25 *µ*g/ml. Bars represent the mean ± standard deviation.

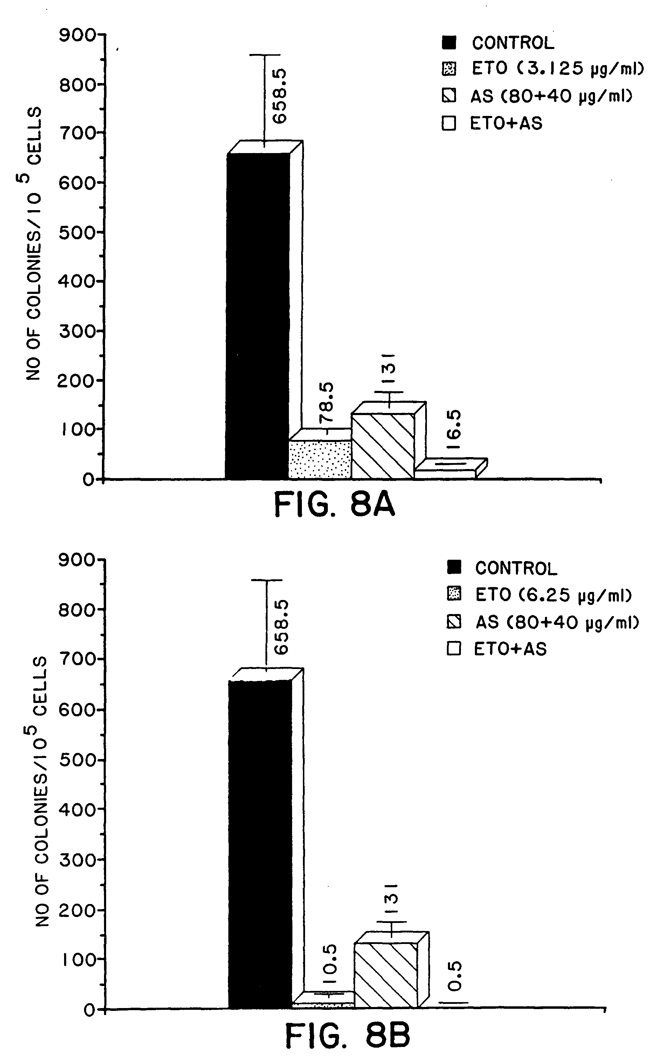

Figs. 8A and 8B are similar to Figs. 7A and 7B, except that etoposide ("ETO") at concentrations of 3.125 (Fig. 8A) and 6.25 (Fig. 8B) *µ*g/ml were substituted for adriamycin.

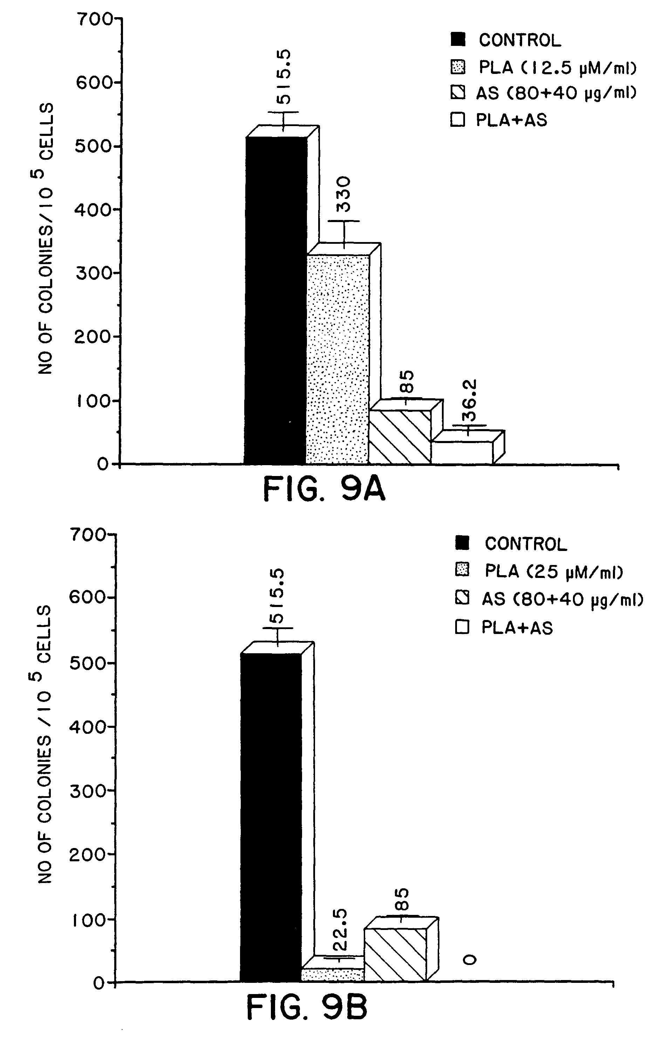

Figs. 9A and 9B are similar to Figs. 7A and 7B, except that cisplatin ("PLA") at concentrations of 12.5 *µ*M/ml (Fig. 9A) and 25 *µ*M/ml (Fig. 9B) were substituted for adriamycin.

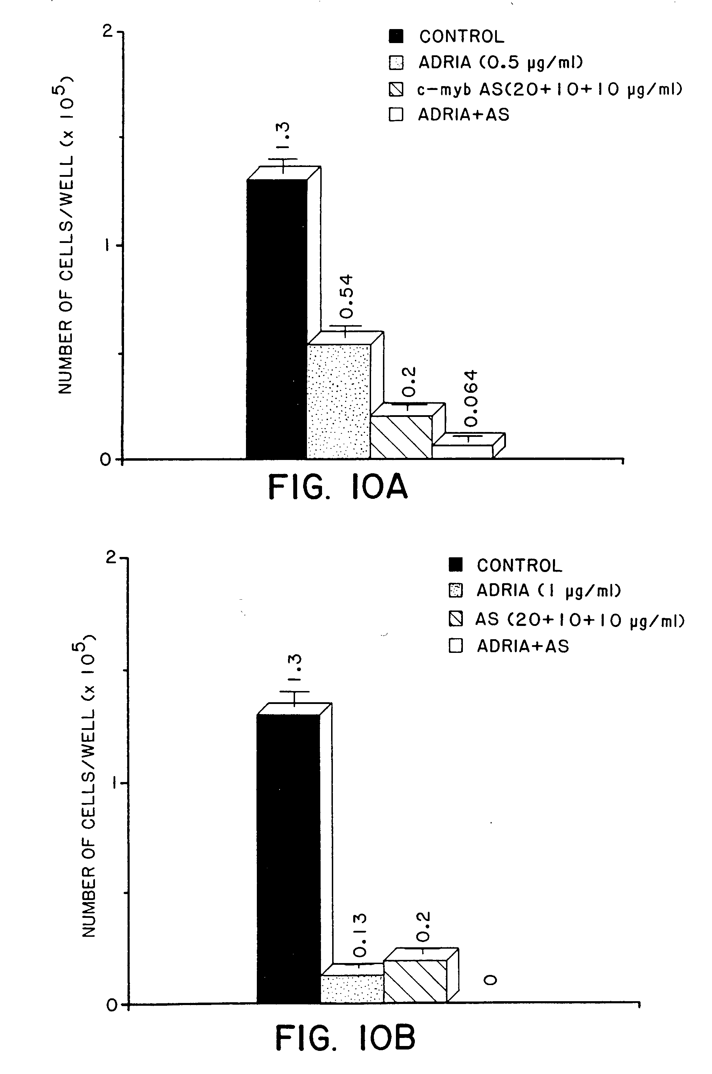

Fig. 10A represents a colony assay of cells of the neuroblastoma cell line LAN-5 incubated with 0.5 *µ*g/ml adriamycin ("ADRIA"); 80 *µ*g/ml for 18 hours (followed by 40 *µ*g/ml for the next 24 hours) of an 18-mer antisense oligonucleotide (SEQ ID NO:5) ("AS") complementary to a portion of c-myb mRNA beginning with translation initiation codon and extending downstream therefrom; the same concentration of the corresponding sense oligomer (SEQ ID NO:6) as a control ("Control"); and the combination of adriamycin (0.5 *µ*g/ml) and antisense oligomer (80 *µ*g/ml for 18 hours, followed by 40 *µ*g/ml for the next 24 hours) ("ADRIA+AS"). Fig. 10B is similar to Fig.10A, except that the concentration of adriamycin is increased to 1 *µ*g/ml. Bars represent the mean ± standard deviation.

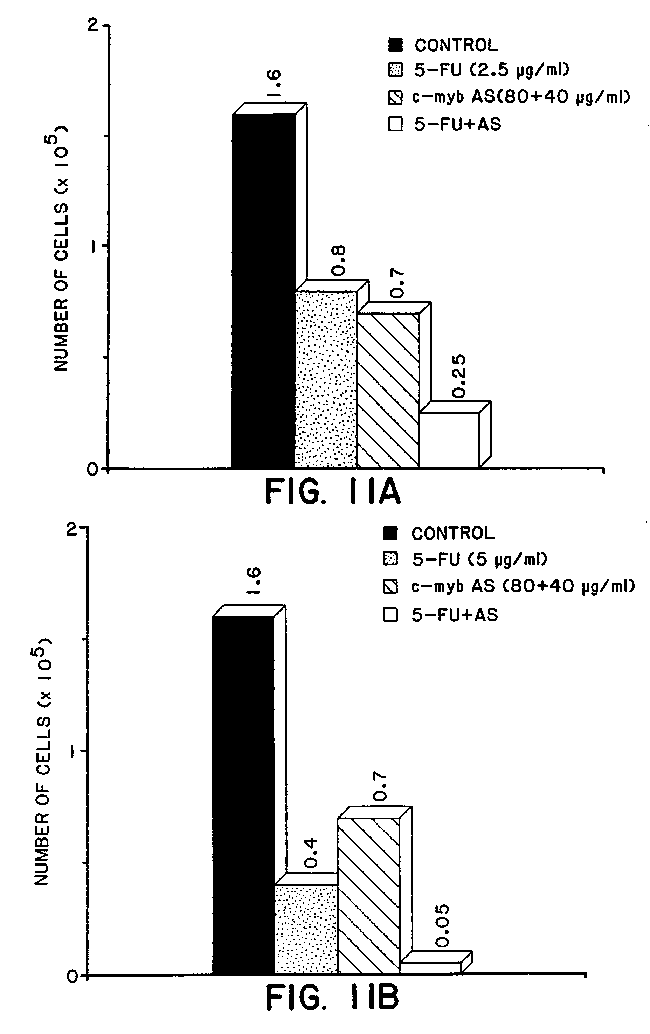

Fig. 11A represents a colony assay of cells of the colorectal carcinoma cell line LOVO incubated with 2.5 *µ*g/ml 5-fluorouracil ("5-FU") ; 80 *µ*g/ml for 18 hours (followed by 40 *µ*g/ml for the next 24 hours) of c-myb antisense oligonucleotide (SEQ ID NO:5); the same concentration of the corresponding sense oligomer (SEQ ID NO:6) as a control ("Control"); and the combination of 5-FU (2.5 *µ* g/ml) and antisense oligomer (80 *µ*g/ml for 18 hours, followed by 40 *µ*g/ml for the next 24 hours) ("5-FU+AS"). Fig. 11B is similar to Fig.11A, except that the concentration of adriamycin is increased to 5 *µ*g/ml. Bars represent the mean ± standard deviation.

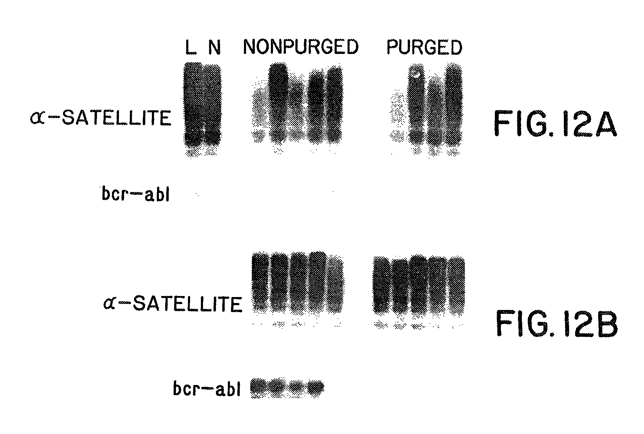

Fig. 12 represents the RT-PCR detection of human α-satellite DNA and bcr-abl mRNA in colonies of marrow cells of sublethally irradiated SCID mice after injection with a 1:1 mixture of normal human A⁻T⁻MNC and CML-BC cells untreated ("NONPURGED") or pretreated ("PURGED") with a combination of mafosfamide (25 *µ*g/ml) and antisense oligonucleotide (SEQ ID NO:1; 80 *µ*g/ml at time zero, followed by 40 *µ*g/ml at 18 hours, and 40 *µ*g/ml at 40 hours). Colonies of CML-BC leukemia (L) or normal bone marrow (N) cells served as positive and negative controls for bcr-abl mRNA detection.

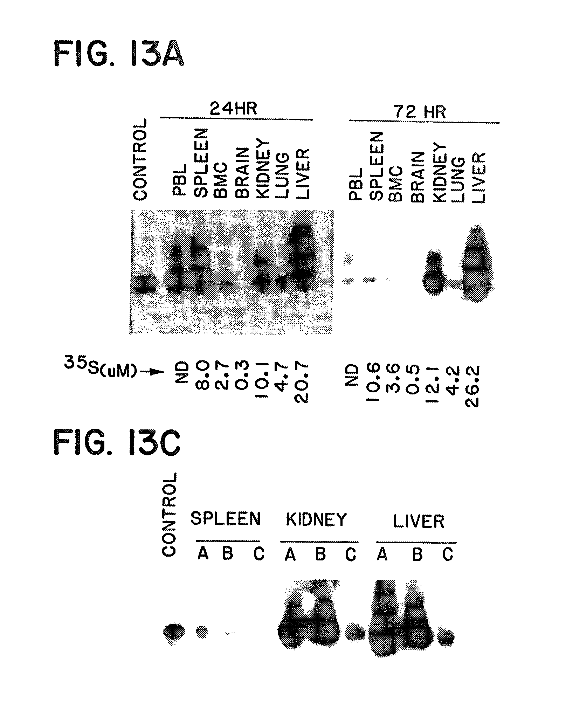

Fig. 13A is a blot of the distribution of bcr-abl (b2a2) ³⁵S-labelled phosphorothioate antisense oligonucleotide in the peripheral blood lymphocytes ("PBL), spleen, bone marrow cells ("BMC"), kidney, lung and liver of mice sacrificed at 24 hours and 72 hours following completion of antisense oligonucleotide therapy (1 mg/day antisense oligonucleotide for 9 consecutive days). The ³⁵S-labelled oligonucleotide concentration in each gram of tissue is indicated, ³⁵S(*µ*M).

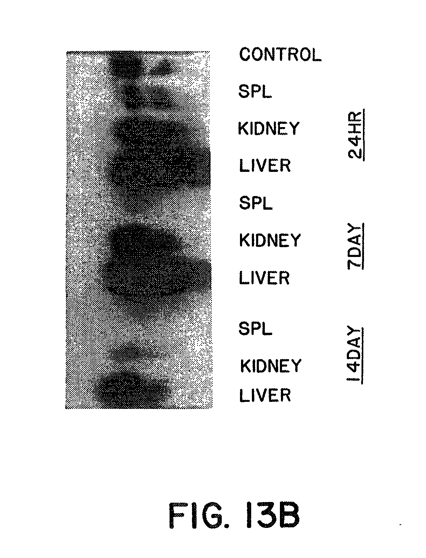

Fig. 13B is similar to Fig. 13A, and shows the phospohorothioate antisense oligonucleotide distribution in the spleen ("SPL"), kidney and liver at 24 hours, 7 days and 14 days post completion of antisense oligonucleotide therapy (1 mg/day for 9 consecutive days).

Fig. 13C is similar to Fig. 13A, and represents the relative concentration of antisense oligonucleotide in nuclear (A), cytoplasmic (B) and membrane (C) fractions from the spleen, kidney and liver of a mouse sacrificed at 24 hours after completion of antisense oligonucleotide therapy (1 mg/day for 9 consecutive days).

### Detailed Description of the Invention

It has been found that when an antisense oligonucleotide which interferes with the expression of a selected oncogene or proto-oncogene (hereinafter collectively "target gene") is combined with other anti-cancer chemotherapeutic agents, the dosage of the latter may be effectively curtailed to a level which spares more normal cells but yet allows efficient elimination of neoplastic cells. Thus, at concentrations which separately do not eliminate all neoplastic cells, elimination of virtually all neoplastic cells expressing the target gene may be achieved by combining an appropriate antisense oligonucleotide with an effective non-antisense chemotherapeutic agent. The result is the highly efficient purging of neoplastic cells with a greatly lessened impact on normal cells. The reduced dosage of the non-antisense agent made possible through combination with the antisense oligonucleotide results in the sparing of more normal cells than would be achieved with the non-antisense agent alone.

The invention is particularly adapted as a method of bone marrow purging wherein the antisense and non-antisense therapeutic agents are used to cleanse aspirated bone marrow of neoplastic cells prior to autologous transplant. The combination of agents is more efficient in the elimination of neoplastic cells at concentrations which are substantially less toxic to normal hematopoietic cells populating the marrow. It is therefore one particular object of the invention to provide a bone marrow purging procedure that will optimize the killing of neoplastic cells, particularly leukemia cells, while sparing normal hematopoietic cells. The combination of a non-antisense chemotherapeutic agent and a tumor-specific antisense oligonucleotide is highly effective in killing leukemic cells and in sparing a much higher number of normal progenitor cells compared to high-dose chemotherapy alone.

The antisense oligonucleotide component of the combination comprises any oligonucleotide which has a nucleotide sequence complementary to a portion of the mRNA of an oncogene or proto-oncogene, the amplification or expression of which is associated with the occurrence of the particular cancerous state. Several such genes are known, and have been sequenced. Antisense oligonucleotides hybridizable to the relevant mRNA may be prepared by known synthetic techniques, based upon the reported cDNA sequences.

Representative target genes for preparation of therapeutic antisense oligonucleotide include the following:
c-myc - Wickstrom et al., Proc. Natl. Acad. Sci. USA 85, 1028-1032 (1988) ; Loke et al., Clin. Res. 36(3), 443A (1988); Holt et al., Cell. Biol. 8, 963-973 (1988); Yokoyama et al., Proc. Natl. Acad. Sci. USA 84, 7363-7367 (1987); Harel-Bellan et al., J. Immunol. 140, 2431-2435 (1988) (inhibition of growth of leukemic cells by antisense oligonucleotides);
L-myc - Nau et al., Nature 318, 69 (1985).
N-myc - Alitalo et al., Advances in Cancer Research 47, 235 (1986).
cyclin D1 - Xiong et al., Cell 65, 691-699 (1991).
c-erbB - Alitalo et al., Advances in Cancer Research 47, 235 (1986).
c-erbB2 - Zhou et al.. Cancer Res. 47, 6123 (1987); Di Fiore et al., Science 237, 178 (1987).
c-fos - Nercola et al., Biochem. Biophys. Res. Comm. 147, 288-294 (1987); Groger et al., Proc. Am. Assoc. Caner Res. 29, 439 (1988) (inhibition of growth of transformed cells by antisense oligonucleotide).
p53 - Shohat et al., Oncogene 1, 277-283 (1987) (inhibition of growth of transformed cells by antisense oligonucleotide); U. S. Patent 5,087,617 (bone marrow purging of malignant cells with antisense oligonucleotide).
c-myb - U.S. Patent 5,098,890 (antisense for treating hematologic neoplasms, including use in bone marrow purging); Melani et al., Cancer Res. 51, 2897-2901 (1991) antisense for inhibiting proliferation of colon cancer cells)
c-abl - International Patent Application WO 91/03260 (antisense for treating myeloproliferative disorders).
c-kit - Ratajczak et al., Proc. Natl. Acad. Sci. USA 89, 1710-1714 (1992) (antisense for inhibiting malignant hematopoietic cell proliferation);
bcr-abl - Szczylick et al., Science 253, 562-565 (1991) (antisense for inhibiting leukemia cell proliferation).
N-ras - Skorski et al., J. Expl. Med. 175(3) 743-750 (1992) (antisense inhibition);
c-Ha-ras- Saison-Behmoaras et al., EMBO 10(5), 1111-1118 (1991); and Daaka et al., Oncogene Res. 5, 267 (1990); Chang et al., Biochemistry 30(34), 8283-8286 (1991) (antisense inhibition).
K-ras - Mukhopadhay et al., Cancer Res. 51(6), 1744-1748 (1991) (antisense inhibition of human lung cancer cells).
PCNA (proliferating cellular nuclear antigen) - Jaskulaski et al., Science 240, 1544-1546 (1988) (antisense inhibition of cell proliferation).
neu - Cooper et al., Nature 311, 29 (1984).

The antisense oligonucleotides for use in the practice of the invention, which are complementary to the target gene's mRNA, may be synthesized by any of the known chemical oligonucleotide synthesis methods. Such methods are generally described, for example, in Winnacker, From Genes to Clones: Introduction to Gene Technology, VCH Verlagsgesellschaft mbH (Ibelgaufts trans. 1987) and Caruthers, Chapter 1, "Synthesis of Oligonucleotides and Oligonucleotide Analogues" in Antisense Inhibitors of Gene Expression, Jack S. Cohen, ed. CRC Press, Inc., Boca Raton, FL (1989) p. 5-24. The antisense oligonucleotides are most advantageously prepared by utilizing any of the commercially available, automated nucleic acid synthesizers. One such device, the Applied Biosystems 380B DNA Synthesizer, utilizes β-cyanoethyl phosphoramidite chemistry.

Based upon the reported nucleotide sequence of DNA complementary to the mRNA transcript of various oncogenes and proto-oncogenes, antisense oligonucleotides hybridizable with any portion of the relevant mRNA transcript may be prepared by oligonucleotide synthesis methods known to those skilled in the art.

While any length oligonucleotide may be utilized in the practice of the invention, sequences shorter than 12 nucleotides, and in particular sequences shorter than 8 nucleotides, may be less specific in hybridizing to the target oncogene/proto-oncogene mRNA, may be more easily destroyed by enzymatic digestion, and may be destabilized by enzymatic digestion. Hence, oligonucleotides having 12 or more nucleotides are generally preferred.

Long sequences, particularly sequences longer than about 50 nucleotides, may be somewhat less effective in inhibiting mRNA translation because of decreased uptake by the target cell. Thus, oligomers of 12-40 nucleotides are preferred, more preferably 15-30 nucleotides, most preferably 18-26 nucleotides. While sequences of 18-21 nucleotides are most particularly preferred for unmodified oligonucleotides, slightly longer chains of up to about 26 nucleotides, are preferred for modified oligonucleotides such as phosphorothioate oligonucleotides, which hybridize less strongly to mRNA than unmodified oligonucleotides.

Oligonucleotides complementary to and hybridizable with any portion of the target mRNA transcript are, in principle, effective for inhibiting translation of the transcript, and capable of inducing the effects herein described. It is believed that translation is most effectively inhibited by blocking the mRNA at a site at or near the initiation codon. Thus, oligonucleotides complementary to the 5'-terminal region of the target mRNA transcript are preferred. The antisense oligonucleotide is preferably directed to a site at or near the initiation codon for protein synthesis. Oligonucleotides complementary to the target mRNA, including the initiation codon (the first codon at the 5' end of the translated portion of the transcript) are preferred.

While antisense oligomers complementary to the 5'- terminal region of the mRNA transcript are preferred, particularly the region including the translation initiation codon, it should be appreciated that useful antisense oligomers are not limited to those complementary to the sequences found in the translated portion of the mRNA transcript, but also includes oligomers complementary to nucleotide sequences contained in, or extending into, the 5'- and 3'-untranslated regions.

The oligonucleotide employed may represent an unmodified or modified oligonucleotide. Thus, oligonucleotides hybridizable to the target mRNA transcript finding utility according to the present invention include not only oligomers of the biologically significant native nucleotides, i.e., A, dA, G, dG, C, dC, T and U, but also oligonucleotide species which have been modified for improved stability and/or lipid solubility. For example, it is known that enhanced lipid solubility and/or resistance to nuclease digestion results by substituting an alkyl group or alkoxy group for a phosphate oxygen in the internucleotide phosphodiester linkage to form an alkylphosphonate oligonucleoside or alkylphosphotriester oligonucleotide. Non-ionic oligonucleotides such as these are characterized by increased resistance to nuclease hydrolysis and/or increased cellular uptake, while retaining the ability to form stable complexes with complementary nucleic acid sequences. The alkylphosphonates in particular, are stable to nuclease cleavage and soluble in lipid. The preparation of alkylphosphonate oligonucleosides is disclosed in U.S. Patent 4,469,863.

Methylphosphonate oligomers can be prepared by a variety of methods, both in solution and on insoluble polymer supports (Agrawal and Riftina, Nucl. Acids Res., 6, 3009-3024 (1979); Miller et al., Biochemistry, 18, 5134-5142 (1979), Miller et al., J. Biol. Chem., 255, 9659-9665 (1980); Miller et al., Nucl. Acids Res., 11, 5189-5204 (1983), Miller et al., Nucl. Acids Res., 11, 6225-6242 (1983), Miller et al., Biochemistry, 25, 5092-5097 (1986); Engels and Jager, Angew. Chem. Suppl. 912 (1982); Sinha et al., Tetrahedron Lett. 24, 877-880 (1983); Dorman et al, Tetrahedron, 40, 95-102 (1984); Jager and Engels, Tetrahedron Lett., 25, 1437-1440 (1984); Noble et al., Nucl. Acids Res., 12, 3387-3404 (1984); Callahan et al., Proc. Natl. Acad. Sci. USA, 83, 1617-1621 (1986); Koziolkiewicz et al., Chemica Scripta, 26, 251-260 (1986); Agrawal and Goodchild, Tetrahedron Lett., 38, 3539-3542 (1987); Lesnikowski et al., Tetrahedron Lett., 28, 5535-5538 (1987); Sarin et al., Proc. Natl. Acad. Sci. USA, 85, 7448-7451 (1988)).

The most efficient procedure for preparation of methylphosphonate oligonucleosides involves use of 5'-O-dimethoxytrityldeoxynucleoside-3'-O-diisopropylmethylphosphoramidite intermediates, which are similar to the methoxy or *β*-cyanoethyl phosphoramidite reagents used to prepare oligodeoxyribonucleotides. The methylphosphonate oligomers can be prepared on controlled pore glass polymer supports using an automated DNA synthesizer (Sarin et al., Proc. Natl. Acad. Sci. USA, 85, 7448-7451 (1988)).

Resistance to nuclease digestion may also be achieved by modifying the internucleotide linkage at both the 5' and 3' termini with phosphoroamidites according to the procedure of Dagle et al., Nucl. Acids Res. 18, 4751-4757 (1990).

Phosphorothioate oligonucleotides contain a sulfur-for-oxygen substitution in the internucleotide phosphodiester bond. Phosphorothioate oligonucleotides combine the properties of effective hybridization for duplex formation with substantial nuclease resistance, while retaining the water solubility of a charged phosphate analogue. The charge is believed to confer the property of cellular uptake via a receptor (Loke et al., Proc. Natl. Acad. Sci. U.S.A. 86, 3474-3478 (1989)).

Phosphorothioate oligodeoxynucleotide are described by LaPlanche, et al., Nucleic Acids Research 14, 9081 (1986) and by Stec et al., J. Am. Chem. Soc. 106, 6077 (1984). The general synthetic method for phosphorothioate oligonucleotides was modified by Stein et al., Nucl. Acids Res., 16, 3209-3221 (1988), so that these compounds may readily be synthesized on an automatic synthesizer using the phosphoramidite approach.

Furthermore, recent advances in the production of oligoribonucleotide analogues mean that other agents may also be used for the purposes described here, e.g., 2'-0-methylribonucleotides (Inove et al., Nucleic Acids Res. 15, 6131 (1987)) and chimeric oligonucleotides that are composite RNA-DNA analogues (Inove et al., FEBS Lett. 215, 327 (1987)).

While inhibition of mRNA translation is possible utilizing either antisense oligoribonucleotides or oligodeoxyribonucleotides, free oligoribonucleotides are more susceptible to enzymatic attack by ribonucleases than oligodeoxyribonucleotides. Hence, oligodeoxyribonucleotides are preferred in the practice of the present invention. Oligodeoxyribonucleotides are further preferred because, upon hybridization with the target mRNA, the resulting DNA-RNA hybrid duplex is a substrate for RNase H, which specifically attacks the RNA portion of DNA-RNA hybrid. Degradation of the mRNA strand of the duplex releases the antisense oligodeoxynucleotide strand for hybridization with additional target gene messages.

In general, the antisense oligonucleotides used in the method of the present invention will have a sequence which is completely complementary to a portion of the target mRNA. Absolute complementarity is not however required, particularly in larger oligomers. Thus, reference herein to a "nucleotide sequence complementary to at least a portion of the mRNA transcript" of a targeted oncogene or proto-oncogene does not necessarily mean a sequence having 100% complementarity with the transcript. In general, any oligonucleotide having sufficient complementarity to form a stable duplex with the relevant mRNA, that is, an oligonucleotide which is "hybridizable", is suitable. Stable duplex formation depends on the sequence and length of the hybridizing oligonucleotide and the degree of complementarity with the targeted region of the message. Generally, the larger the hybridizing oligomer, the more mismatches may be tolerated. More than one mismatch probably will not be tolerated for antisense oligomers of less than about 21 nucleotides. One skilled in the art may readily determine the degree of mismatching which may be tolerated between any given antisense oligomer and the target mRNA sequence, based upon the melting point, and therefore the stability, of the resulting duplex. Melting points of duplexes of a given base pair composition can be readily determined from standard texts, such as Molecular Cloning: A Laboratory Manual, (2nd edition, 1989), J. Sambrook et al., eds., Cold Spring Harbor Laboratory Press.

While oligonucleotides capable of stable hybridization with any region of the target gene's message are within the scope of the present invention, oligonucleotides complementary to a region including the initiation codon are believed particularly effective. Particularly preferred are oligonucleotides hybridizable to a region of the relevant mRNA up to 40 nucleotides upstream (in the 3'→ 5' direction) of the initiation codon or up to 40 nucleotides downstream (in the 5'→3' direction) of that codon.

The non-antisense component of the therapeutic combination may comprise any antineoplastic (anti-cancer) agent useful in the treatment of the particular disease state characterized by the expression of the targeted oncogene/proto-oncogene. The non-antisense component may comprise a single chemical agent. More typically, it will comprise a combination of agents. It is now generally regarded among clinicians that except for a relatively few cancers, such as Burkitt's lymphoma, combination therapy is required to cure all drug-sensitive tumors.

The non-antisense oligonucleotide component may comprise, for example, the following:
antimetabolites - folic acid analogues such as methotrexate and trimetrexate; pyrimidine analogues such as 5-fluorouracil (5-FU), 5-fluorodeoxyuridine, cytosine arabinoside (ara-C) and 5-azacytidine; purine analogues such as 6-mercaptopurine and 6-thioguanine; hydroxyurea; and deoxycoformycin;
alkylating agents - nitrogen mustards; ethylenimine derivatives such as triethylenethiophosoporamide (thio-tepa); alkyl sulfonates such as busulfan; cyclophosphamide; 4-hydroxyperoxycyclophosphoramide (4-HC); mafosfamide; ifosfamide; melphalan; chlorambucil; nitrosoureas such as cyclohexylnitrosourea (CCNU), bis-chloroethylnitrosourea (BCNU) and methylcyclohexylnitrosourea (meCCNU); cis (II) platinum diamminedichloride (cisplatin or platinol);
plant alkaloids - vinca alkaloids such as vinblastine, vincristine and vindesine; epipodophyllotoxins such as etoposide and teniposide;
antitumor antibiotics - bleomycin; anthracyclines such as daunomycin, doxorubicin (adriamycin), epirubicin, idarubicin and esorubicin; mitomycin C; actinomycin D; and mithramycin;
hormones - adrenocorticosteroids such as a prednisone; progestins such as hydroxyprogesterone; androgens such as testosterone; estrogens such as diethylstilbestrol; and anti-estrogens such as tamoxifen;
miscellaneous agents - methylhydrazine derivatives such as dacarbazine and procarbazine; hexamethylmelamine, pentamethylmelamine, mitoxantrone and amsacrine.

The dosage of the non-antisense agent may vary over a wide range. Preferably, the dosage is selected from about 25% to about 50% of the maximum recommended dosage for the drug. The recommended dosage for the major antineoplastic drugs, together with corresponding routes of injection, preferred injection vehicles, infusion duration, dose frequency, acute toxicity and pharmacokinetic data may be found in Cancer: Principles & Practice of Oncology (3rd ed., 1989), Vincent T. DeVita, Jr. et al., eds., J.B. Lippincott Co., Philadelphia, PA, pages 349-395, incorporated herein by reference. The 50-75% reduction in drug dosage achieved through the practice of the invention means that the treatment is concomitantly less toxic to the host.

The following listing from DeVita et al., pages 352 and 354 contains the recommended dosages, corresponding routes of injection, injection vehicles, infusion durations, and dose frequencies for major antineoplastic drugs.

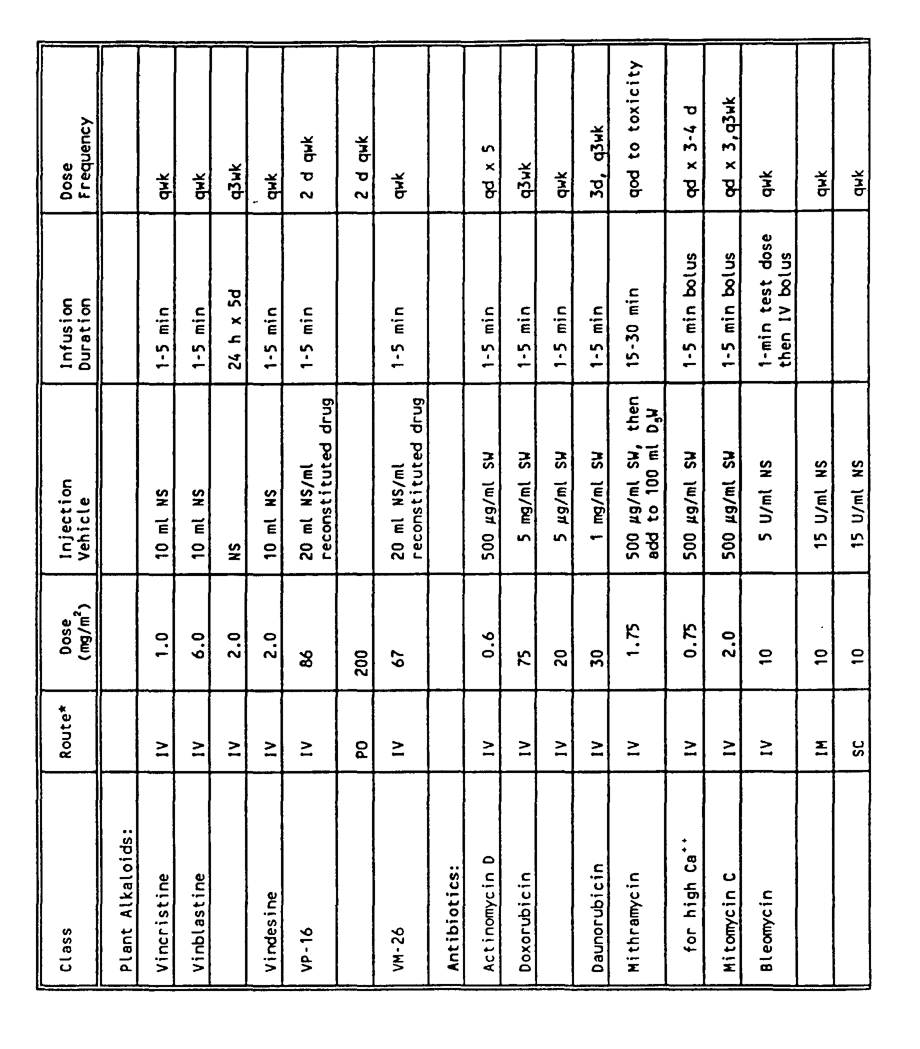

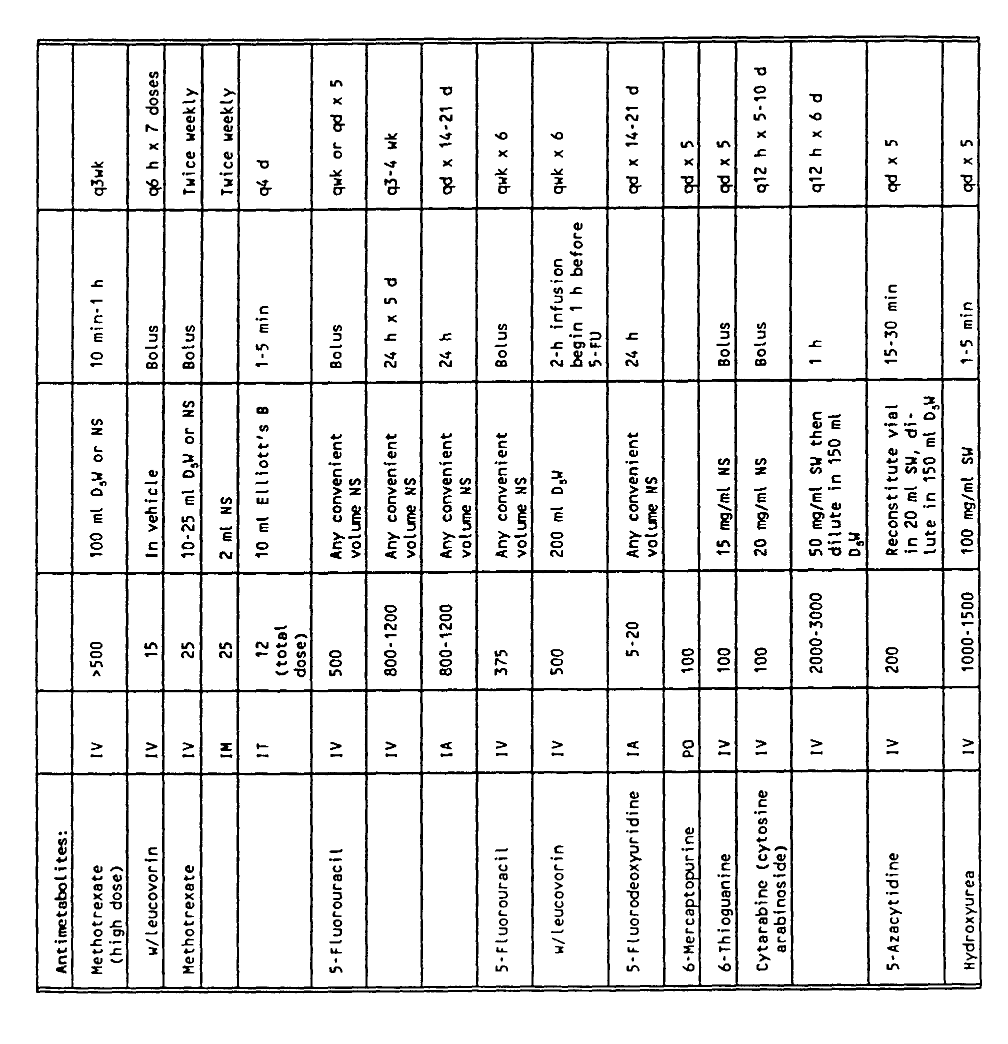

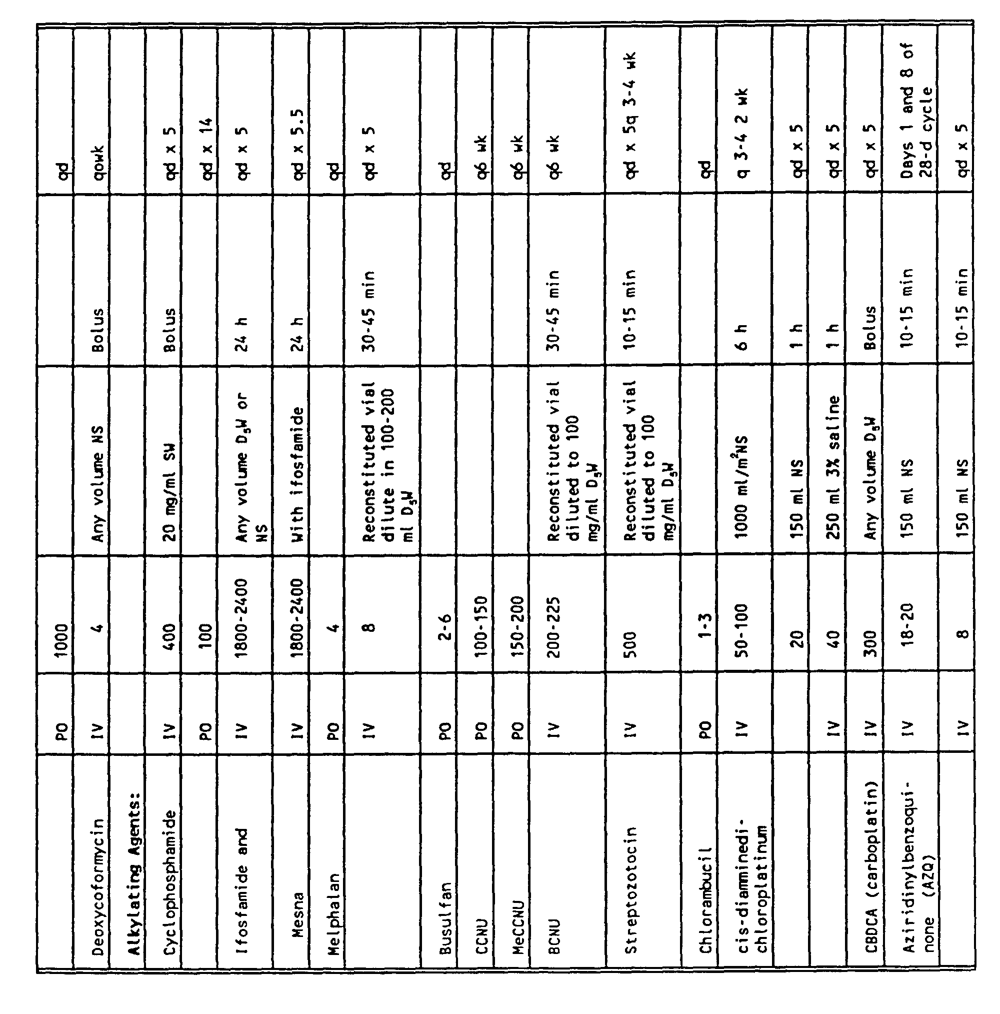

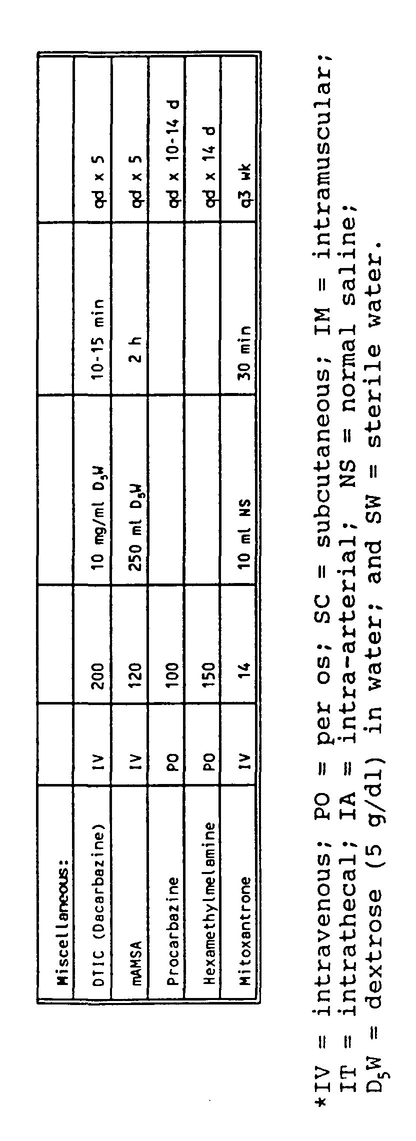

The non-antisense agent may be administered through any of the routes recommended for such agents. Typically, the non-antisense agent will be administered parenterally, such as through intravenous injection or infusion.

While it is preferred that the antisense and non-antisense agents be administered simultaneously, such as in the form of a single pharmaceutical composition, the two agents may also be administered separately, in sequence. While it is presently preferred that both agents are administered through the same route, they may be administered by different routes.

For in vivo or ex vivo use, the antisense oligonucleotide and non-antisense agent may be combined with a pharmaceutical carrier, such as a suitable liquid vehicle or excipient and an optional auxiliary additive or additives. The liquid vehicles and excipients are conventional and commercially available. Illustrative thereof are distilled water, physiological saline, aqueous solution of dextrose, and the like. For in vivo use, the antisense oligonucleotide and non-antisense drug are preferably administered parenterally, most preferably intravenously. The vehicle is designed accordingly. Alternatively, the active agents may be administered subcutaneously via controlled release dosage forms.

In addition to administration with conventional carriers, at least the antisense oligonucleotide component of the combination may be administered by a variety of specialized oligonucleotide delivery techniques. For example, oligonucleotides may be encapsulated in liposomes for therapeutic delivery. The oligonucleotide, depending upon its solubility, may be present both in the aqueous layer and in the lipidic layer, or in what is generally termed a liposomic suspension. The hydrophobic layer, generally but not exclusively, comprises phospholipids such as lecithin and sphingomyelin, steroids such as cholesterol, ionic surfactants such as diacetylphosphate, stearylamine, or phosphatidic acid, and/or other materials of a hydrophobic nature. Oligonucleotides have been successfully encapsulated in unilamellar liposomes.

Reconstituted Sendai virus envelopes have been successfully used to deliver RNA and DNA to cells. Arad et al., Biochem. Biophy. Acta. 859, 88-94 (1986).

The oligonucleotide component of the therapeutic combination may be conjugated to poly(L-lysine) to increase cell penetration. Such conjugates are described by Lemaitre et al., Proc. Natl. Acad. Sci. USA, 84, 648-652 (1987). The procedure requires that the 3'-terminal nucleotide be a ribonucleotide. The resulting aldehyde groups are then randomly coupled to the epsilon-amino groups of lysine residues of poly(L-lysine) by Schiff base formation, and then reduced with sodium cyanoborohydride. This procedure converts the 3'-terminal ribose ring into a morpholine structure.

The oligonucleotide may be conjugated for therapeutic administration to ligand-binding molecules which recognize cell-surface molecules, such as according to International Patent Application WO 91/04753. In particular, transferrin-polylysine-oligonucleotide complexes may be prepared for uptake by cells expressing high levels of transferrin receptor. The preparation of such complexes as carriers of oligonucleotide uptake into cells is described by Wagner et al., Proc. Natl. Acad. Sci. USA 87, 3410-3414 (1990). Inhibition of leukemia cell proliferation by transferrin receptor-mediated uptake of c-myb antisense oligonucleotides conjugated to transferrin has been demonstrated by Citro et al., Proc. Natl. Acad. Sci. USA 89, 7031-7035 (1992).

The disorders treatable with the combination therapy according to the practice of the invention include neoplastic diseases wherein the target gene is amplified, that is, the copy number of the gene is enhanced above normal levels. The disorders treatable also include those disorders characterized by the activation of the target gene's expression, signalled by the appearance of the relevant mRNA transcripts and/or the corresponding protein product. Oncogene or proto-oncogene amplification or expression may be assayed by conventional probing techniques, such as described in Molecular Cloning: A Laboratory Manual (2nd ed. 1989). Briefly, tumor cell DNA is digested with a restriction enzyme, e.g. PstI, and fractionated by electrophoresis on a 0.8% agarose gel for Southern blot analysis. After transfer to the appropriate membrane, the tumor DNA is hybridized to a radiolabelled DNA fragment of the target gene. As a control, DNA of contiguous normal tissue is also analyzed. Amplification is assessed relative to the copy number for the same gene in normal cells from the contiguous normal tissue. In a similar fashion, the level of target gene expression is determined by probing total cellular RNA from tumor cells with a complementary probe for the relevant mRNA. Total RNA from the tumor cells is fractionated in a glyoxal/agarose gel, transferred to nylon and hybridized to an appropriately labelled nucleic acid probe for the target mRNA. The number of relevant mRNA transcripts found in the tumor cells is compared to that found in normal cells from the same tissue.

At least a 10-fold amplification of the target gene in patient neoplastic cells over normal cells from the same tissue would be indicative that the patient's disease would be susceptible to treatment by antisense oligonucleotide. Similarly, an at least 10-fold increase in that gene's expression in neoplastic cells over expression in normal cells from the same tissue would indicate that the disease would respond to antisense treatment. These thresholds are based upon correlations between the level of gene amplification/expression and the extent of the disease state for various oncogenes. See, for example, Slamon et al., Science 235, 177-182 (1988) and Science, 244, 707-712 (1989) (correlation between erb-b2 amplification/expression and breast or ovarian cancer); Alitalo et al., Advances in Cancer Research, 47, 235-282 (1986).

For each disease, therapy comprises administration of an antisense oligonucleotide complementary to the mRNA of the targeted gene, in combination with a non-antisense chemotherapeutic agent. The latter may be selected according to the recommendations in the literature and standard oncology treatises, such as Cancer: Principles and Practice of Oncology (3rd ed. 1989), V.T. DeVait, Jr. et al., eds. J.B. Lippincott Co., Philadelphia, PA. Thus, for CML, for example, treatment comprises administering a combination of bcr-abl antisense oligonucleotide and one or more non-oligonucleotide drugs, e.g., such as hydroxyurea for chronic phase treatment, or vincristine plus prednisone followed by L-asparaginase for acute phase treatment. Maintenance with methotrexate and 6-mercaptopurine may also be used.

The following is a partial list of disease conditions which have been linked to the amplification or expression of various oncogenes or proto-oncogenes.
c-myc - leukemia, lymphoma, breast cancer, myeloma, neuroepithelioma and lung cancer;
N-myc - neuroectodermal tumors (neuroblastoma and neuroepithelioma), and lung cancer;
L-myc - lung cancer;
c-erbB(EGFR) - glioblastoma, leukemia, lung cancer; and squamous cell head and neck cancer;
c-erbB2(HER2) - ovarian and mammary carcinoma;
c-fos - osteoblastoma;
p53 - leukemia;
c-abl - leukemia and lymphoma;
cyclin D1 - melanoma, neuroectodermal tumors, esophageal cancer, breast cancer, squamous cell cancers, parathyroid adenomas, leukemia and lymphoma;
c-myb - leukemia, lymphoma, melanoma, colorectal carcinoma, neuroectodermal tumors;
c-abl - leukemia and lymphoma;
c-kit - leukemia and lymphoma;
bcr-abl - CML and acute lymphocytic leukemia;
N-ras - leukemia;
c-Ha-ras and K-ras - prostate cancer, bladder carcinoma, breast cancer, lung cancer, myeloma, colon cancer and other tumors of epithelial origin;
neu - breast and ovarian cancer.

A preferred method of administration of both the antisense oligonucleotide and the non-antisense drug comprises either regional or systemic perfusion, as is appropriate. According to a method of regional perfusion, the afferent and efferent vessels supplying the extremity containing the lesion are isolated and connected to a low-flow perfusion pump in continuity with an oxygenator and a heat exchanger. The iliac vessels may be used for perfusion of the lower extremity. The axillary vessels are cannulated high in the axilla for upper extremity lesions. The active agent(s) is/are added to the perfusion circuit, and the perfusion is continued for an appropriate time period, e.g., one hour. Perfusion rates of from 100 to 150 ml/minute may be employed for lower extremity lesions, while half that rate should be employed for upper extremity lesions. Systemic heparinization may be used throughout the perfusion, and reversed after the perfusion is complete. This isolation perfusion technique permits administration of higher doses of chemotherapeutic agent than would otherwise be tolerated upon infusion into the arterial or venous systemic circulation.

For systemic infusion, the antisense oligonucleotide and/or non-antisense chemotherapeutic agent are preferably delivered via a central venous catheter, which is connected to an appropriate continuous infusion device. Indwelling catheters provide long term access to the intravenous circulation for frequent administration of drugs over extended time periods. They are generally surgically inserted into the external cephalic or internal jugular vein under general or local anesthesia. The subclavian vein is another common site of catheterization. The infuser pump may be external, or may form part of an entirely implantable central venous system such as the INFUSAPORT system available from Infusaid Corp., Norwood, MA and the PORT-A-CATH system available from Pharmacia Laboratories, Piscataway, NJ. These devices are implanted into a subcutaneous pocket under local anesthesia. A catheter, connected to the pump injection port, is threaded through the subclavian vein to the superior vena cava. The implant contains a supply of active agent in a reservoir which may be replenished as needed by injection of additional drug from a hypodermic needle through a self-sealing diaphragm in the reservoir. Completely implantable infusers are preferred, as they are generally well accepted by patients because of the convenience, ease of maintenance and cosmetic advantage of such devices.

At least the antisense oligonucleotide may also be administered locally, as contrasted to regional or systemic administration. Local administration of polynucleotides have been carried out by direct injection into muscle. Local administration of oligonucleotides may be particularly useful in treating neuroectodermal tumors, esophageal tumors and melanoma. For treatment of esophageal tumors, a pharmaceutical preparation of antisense oligonucleotide may be delivered locally to the tumor site by means of a catheter. Such catheters have been used to deliver drugs for local cardiovascular treatment and can be adapted for use in delivering drug directly to esophageal lesions. For treatment of melanoma, the oligonucleotides may be delivered by skin infiltration. Methods for delivering therapeutic oligonucleotide and polynucleotides by local infiltration are known to those skilled in the art.

As an alternative to treatment with exogenous oligonucleotide, the antisense oligonucleotide of the therapeutic combination may be synthesized in situ at the disease lesion by local treatment of the targeted neoplastic cells with a vector containing an artificially-constructed gene comprising a transcriptional promotor and target gene DNA in inverted orientation. The DNA for insertion into the artificial gene in inverted orientation comprises cDNA which may be prepared, for example, by reverse transcriptase polymerase chain reaction from RNA using primers derived from the reported cDNA sequence of the target gene. Upon transcription, the inverted gene segment, which is complementary to at least a portion of the target gene's mRNA, is produced in situ in the targeted cell. The endogenously produced RNA hybridizes to the relevant mRNA, resulting in interference with the target gene's function and inhibition of the proliferation of the targeted neoplastic cell. At the same time, the non-antisense agent is delivered to the lesion locally, or is given systemically.

The promotor segment of the artificially-constructed gene serves as a signal conferring expression of the inverted oncogene/proto-oncogene sequence which lies downstream thereof. It will include all of the signals necessary for initiating transcription of the sequence. The promotor may be of any origin as long as it specifies a rate of transcription which will produce sufficient antisense mRNA to inhibit the expression of the target gene, and therefore the proliferation of the tumor cells. Preferably, a highly efficient promotor such as a viral promotor is employed. Other sources of potent promotors include cellular genes that are expressed at high levels. The promotor segment may comprise a constitutive or a regulatable promotor. A typical construct will utilize the SV40 promotor.

The artificial gene may be introduced by any of the methods described in U.S. Patent 4,740,463, incorporated herein by reference. One technique is transfection, which can be done by several different methods. One method of transfection involves the addition of DEAE-dextran to increase the uptake of the naked DNA molecules by a recipient cell. See McCutchin, J.H. and Pagano, J.S., J. Natl. Cancer Inst. 41, 351-7 (1968). Another method of transfection is the calcium phosphate precipitation technique which depends upon the addition of Ca⁺⁺ to a phosphate-containing DNA solution. The resulting precipitate apparently includes DNA in association with calcium phosphate crystals. These crystals settle onto a cell monolayer; the resulting apposition of crystals and cell surface appears to lead to uptake of the DNA. A small proportion of the DNA taken up becomes expressed in a transfectant, as well as in its clonal descendants. See Graham, F.L. and van der Eb, A.J., Virology 52, 456-467 (1973) and Virology 54, 536-539 (1973).

Transfection may also be carried out by cationic phospholipid-mediated delivery. In particular, polycationic liposomes can be formed from N-[1-(2,3-dioleyloxy)propyl]-N,N,N-trimethylammonium chloride (DOTMA). See Felgner et al., Proc. Natl. Acad. Sci. USA 84, 7413-7417 (1987) (DNA-transfection); Malone et al., Proc. Natl. Acad. Sci. USA, 86, 6077-6081 (1989) (RNA-transfection); Zhu et al., Science 261, 209-211 (1993).

Alternatively, the artificially-constructed gene can be introduced in to cells, in vitro or in vivo, via a transducing viral vector. See Tabin et al., Mol. Cel. Biol. 2, 426-436 (1982). Use of a retrovirus, for example, will infect a variety of cells and cause the artificial gene to be inserted into the genome of infected cells. Such infection could either be done with the aid of a helper retrovirus, which would allow the virus to spread through the organism, or the antisense retrovirus could be produced in a helper-free system, such as ψ2-like cells (See Mann et al., Cell 33, 153-160, 1983) that package amphotropic viruses. A helper-free virus might be employed to minimize spread throughout the organism. Viral vectors in addition to retroviruses can also be employed, such as papovaviruses, SV40-like viruses, or papilloma viruses. The use of retroviruses for gene transfer has been reviewed by Eglitis and Anderson, BioTechniques 6, 608-614 (1988).

Vesicle fusion could also be employed to deliver the artificial gene. Vesicle fusion may be physically targeted to the tumor tissue if the vesicle were appropriately designed to be taken up by the cells containing the target gene. Such a delivery system would be expected to have a lower efficiency of integration and expression of the artificial gene delivered, but would have a higher specificity than a retroviral vector. A combination strategy of targeted vesicles containing papilloma virus or retrovirus DNA molecules might provide a method for increasing the efficiency of expression of targeted molecules.

Still another alternative is to introduce the artificial gene via micro-injection. See for example, Laski et al., Cell, 1982.

Particulate systems and polymers for in vitro and in vivo delivery of polynucleotides was extensively reviewed by Felgner in Advanced Drug Delivery Reviews 5, 163-187 (1990). Techniques for direct delivery of purified genes in vivo, without the use of retroviruses, has been reviewed by Felgner in Nature 349, 351-352 (1991). Such methods of direct delivery of polynucleotides may be utilized for local delivery of either exogenous antisense oligonucleotide or artificially-constructed genes producing the antisense oligonucleotide in situ.

Recently, Wolf et al. demonstrated that direct injection of non-replicating gene sequences in a non-viral vehicle is possible. See Science, 247, 1465-1468 (1990). DNA injected directly into mouse muscle did not integrate into the host genome, and plasmid essentially identical to the starting material was recovered from the muscle months after injection. Interestingly, no special delivery system is required. Simple saline or sucrose solutions are sufficient to delivery DNA and RNA.

The antisense oligonucleotide and non-antisense drug may be administered to the patient in the form of an appropriate pharmaceutical composition, either in combination or separately. Alternatively, the active agents may be administered ex vivo, to cells harvested from the patient. The antisense oligonucleotide and non-antisense agent may be contacted with the harvested cells sequentially or simultaneously. Thus, according to a preferred embodiment of the invention, the combination is utilized as a bone marrow purging agent for in vitro cleansing of the patient's bone marrow contaminated by leukemic or other neoplastic cells. The combination is believed useful for purging in either allogeneic or autologous bone marrow transplantation.

While it is contemplated that the principal application of the present invention will be for bone marrow purging for leukemia patients, many other neoplasms, such as neuroblastoma, melanoma and breast cancer, may be substantially metastatic, particularly in advanced stages, and would benefit from bone marrow purging. In particular, malignant cells may metastasize to the bone marrow. Patients with disseminated disease may have bone marrow metastases.

According to a method for bone marrow purging, bone marrow is harvested from a donor by standard operating room procedures from the iliac bones of the donor. Methods of aspirating bone marrow from donors are well-known in the art. Examples of apparatus and processes' for aspirating bone marrow from donors are disclosed in U.S. Patents 4,481,946 and 4,486,188, incorporated herein by reference. Sufficient marrow is withdrawn so that the recipient, who is either the donor (autologous transplant) or another individual (allogeneic transplant), may receive from about 4 x 10⁸ to about 8 x 10⁸ processed marrow cells per kg of bodyweight. This generally requires aspiration of about 750 to about 1000 ml of marrow. The aspirated marrow is filtered until a single cell suspension, known to those skilled in the art as a "buffy coat" preparation, is obtained. This suspension of leukocytes is treated with antisense oligonucleotides in a suitable carrier, advantageously in a concentration of about 50-100 *µ* g/ml. Alternatively, the leucocyte suspension may be stored in liquid nitrogen using standard procedures known to those skilled in the art until purging is carried out. The purged marrow can be stored frozen in liquid nitrogen until ready for use. Methods of freezing bone marrow and biological substances are disclosed, for example, in U.S. Patents 4,107,937 and 4,117,881.

Other methods of preparing bone marrow for treatment with antisense oligonucleotide may be utilized, which methods may result in even more purified preparations of hematopoietic cells than the aforesaid buffy coat preparation.

Either prior to, or simultaneously with, or subsequent to treatment with antisense oligonucleotide, the bone marrow cells are similarly treated with the non-antisense agent. The latter is used according to existing protocols for bone marrow purging, except to the extent that the dosage may be substantially reduced from the concentrations typically recommended for this purpose. Dosage reductions of from 2- to 4-fold are believed possible, depending upon the drug. Thus, for example, where the marrow purging concentration of mafosfamide when administered alone is 50-100 *µ*g/ml, combining mafosfamide with bcr-abl antisense oligonucleotide allows the dosage to be reduced to as low as 12.5-25 *µ*g/ml. As a consequence of this reduced dosage, a significantly greater number of normal bone marrow cells are spared for repopulating the marrow, but without sacrifice to purging efficiency.

The appropriate concentration of the non-antisense agent may be determined by first exposing the patient cells to increasing concentrations of the non-antisense agent in a clonogenic assay in advance of bone marrow purging. The maximum tolerated dosage is determined, that is, the concentration which achieves essentially complete killing of neoplastic cells but which spares at least a modest number of normal cells. The concentration of the non-antisense agent is then decreased 2- to 4-fold, or possibly more, from the maximum tolerated dosage determined by the clonogenic assay. The appropriate antisense oligonucleotide is then utilized for bone marrow purging in combination with the reduced concentration of non-antisense agent.

Where the bone marrow purging is carried out as an anti-leukemic treatment, one or more hematopoietic growth factors may be added to the aspirated marrow or buffy coat preparation to stimulate growth of hematopoietic neoplasms, and thereby increase their sensitivity to the toxicity of the antisense oligonucleotide. Such hematopoietic growth factors include, for example, IL-3 and granulocyte macrophage colony stimulating factor (GM-CSF). The recombinant human versions of such growth factors are advantageously employed.

After treatment with the antisense and non-antisense agents, the cells to be transferred are washed with autologous plasma or buffer to remove unincorporated drug. The washed cells are then infused back into the patient. Other methods for bone marrow purging utilizing antisense oligonucleotide are disclosed in U.S. Patent 5,087,617, incorporated herein by reference.

For in vivo administration, the amount of antisense oligonucleotide and non-antisense agent may vary depending on the nature and extent of the neoplasm, the particular oligonucleotide utilized, and other factors. The actual dosage of each drug administered may take into account the size and weight of the patient, whether the nature of the treatment is prophylactic or therapeutic in nature, the age, health and sex of the patient, the route of administration, whether the treatment is regional or systemic, and other factors.

For the antisense oligonucleotide, sufficient oligomer should be administered to achieve an intracellular concentration of from about 1 to about 100 *µ*g/ml, preferably from about 10 *µ*g/ml to about 100 *µ*g/ml, most preferably from about 20 *µ*g/ml to about 60 *µ*g/ml. The patient should receive a sufficient daily dosage of antisense oligonucleotide to achieve these concentrations of drug. The daily dosage may range from about 0.1 to 1,000 mg oligonucleotide per day, preferably from about 100 to about 700 mg per day. Greater or lesser amounts of oligonucleotide may be administered, as required. Those skilled in the art should be readily able to derive appropriate dosages and schedules of administration to suit the specific circumstance and needs of the patient.

It is believed that a course of treatment may advantageously comprise infusion of the recommended daily dose of oligonucleotide for a period of from about 3 to about 28 days, more preferably from about 7 to about 10 days. Those skilled in the art should readily be able to determine the optimal dosage in each case. For modified oligonucleotides, such as phosphorothioate oligonucleotides, which have a half life of from 24 to 48 hours, the treatment regimen may comprise dosing on alternate days.

A daily oligonucleotide dosage of 250-300 mg will provide an extracellular oligonucleotide concentration of 1.5-2.5 *µ*M which, based upon in vitro studies, is an effective concentration. Thus, for an about 70 kg adult human being, a daily dose of about 250-350 mg oligonucleotide is believed sufficient to achieve an effective extracellular concentration. For children, the daily dosage is reduced proportionately according to the weight of the patient.

For ex vivo antineoplastic application, such as, for example, in bone marrow purging, the antisense oligonucleotides and non-antisense agent may be administered in amounts effective to kill neoplastic cells. Such amounts may vary depending on the extent to which malignant cells exist in the bone marrow, the identity of the particular oligonucleotide and non-antisense drug utilized, the relative sensitivity of the neoplastic cells to the non-antisense drug and oligonucleotide, and other factors. For the antisense oligonucleotide, concentrations from about 10 to 200 *µ*g/ml per 10⁶ or 10⁷ cells may be employed, preferably from about 40 to 150 *µ*g/ml per 10⁵ or 10⁶ cells. Supplemental dosing of the same or lesser amounts of oligonucleotide are advantageous to optimize the treatment. Thus, for purging bone marrow containing 2 x 10⁷ cells per ml of marrow volume, dosages of from about 2 to 40 mg antisense per ml of marrow may be effectively utilized, preferably from about 8 to 24 mg/ml. Greater or lesser amounts of oligonucleotide may be employed.

The non-antisense agent is used in bone marrow purging in a concentration of from about 25% to about 50% of the otherwise literature-recommended dosage when used alone. Preferred agents for bone marrow purging include, for example, 4-HC, mafosfamide, ara-C, BCNU, busulfan, etoposide, 5-FU, doxorubicin, cisplatin, or combination thereof. Bone marrow purging may be used, in particular, for treatment of hematologic neoplasms such as acute nonlymphoblastic leukemia, acute lymphoblastic leukemia, chronic myelogenous leukemia and lymphoma.

The effectiveness of treatment may be assessed by routine methods which are used for determining whether or not remission of neoplastic disease has occurred. Such methods generally depend upon some combination of morphological, cytochemical, cytogenetic, immunologic and molecular analyses. In addition, remission can be assessed genetically by probing the level of expression of the target oncogene/protooncogene. The reverse transcriptase polymerase chain reaction methodology can be used to detect even very low numbers of mRNA transcript.

Typically, therapeutic success is assessed by the decrease in the extent of the primary and any metastatic diseases lesions. For solid tumors, decreasing tumor size is the primary indicia of successful treatment. Neighboring tissues should be biopsied to determine the extent to which metastasis has occurred. Tissue biopsy methods are known to those skilled in the art. For non-solid tumors, i.e. the leukemias, treatment is monitored primarily by histological examination of the bone marrow for surviving leukemic cells. However, a significant number of leukemic cells may still exist when marrow examination provides normal results. For this reason, more recent methods for detecting leukemic cells have focused on detecting the presence of the gene for the relevant oncogene, or its corresponding mRNA, in cells of the bone marrow as a more sensitive test. See for example the following U.S. Patents: 4,681,840, 4,857,466 and 4,874,853. The presence of even a few copies of the target oncogene can be effectively detected by amplification using reverse transcriptase polymerase chain reaction technology. For a detailed discussion of methods for assessing therapeutic success see, for example, Cancer: Principles & Practice of Oncology, edited by V. T. DeVita, S. Hellman and S.A. Rosenberg, J.B. Lippincott Company, Philadelphia, PA (3rd ed., 1989), incorporated herein by reference. Methods for diagnosing and monitoring the progress of neoplastic disorders vary depending upon the nature of the particular disease.

According to one preferred embodiment, the invention comprises in vivo or ex vivo treatment of Ph¹-positive leukemias, that is, leukemias characterized by the chromosomal abnormality known as the Philadelphia or Ph¹ chromosome. At the molecular level, the most notable feature is the translocation of the proto-oncogene c-abl from the long arm of chromosome 9 to the breakpoint cluster region (bcr) on chromosome 22, resulting in the formation of bcr-abl hybrid genes. The break occurs near the end of the long arm of chromosome 9 (band 9q34) and in the upper half of chromosome 22 (band 22q11).

The c-abl proto-oncogene normally encodes a protein with tyrosine kinase activity. This activity is augmented in cells carrying bcr-abl hybrid genes. The gene located at the breakpoint on chromosome 22 is called bcr because the break in chromosome 22 in CML occurs in a very small 5.8-kilobase (kb) segment (breakpoint cluster region) of the gene on chromosome 22. Two alternative first exons of the c-abl oncogene exist, namely exon 1a and exon 1b, which are spliced to the common splice acceptor site, exon 2. As a result of this configuration, at least two major c-abl messages are transcribed, differing in their 5' regions. (Shtivelman et al., Cell 47, 277 (1986); Bernards et al., Mol. Cell. Biol. 7, 3231 (1987); Fainstein et al., Oncogene 4, 1477-1481 (1989)). If exon 1b is used, the mRNA is 7.0 kb. If exon 1a is used, the mRNA is 6.0 kb. Each of exons 1a and 1b are preceded by a transcriptional promotor. The 9;22 translocation in CML results in the abnormal juxtaposition of abl sequences adjacent to bcr sequences. The fusion leads to an 8.5 kb chimeric mRNA consisting of 5' BCR sequences and 3' abl sequences. The chimeric message is in turn translated into a larger chimeric abl protein (210 kDa) that has increased tyrosine kinase activity (Konopka et al., Cell 37, 1035 (1984); Kloetzer et al., Virology 140, 230 (1985); Konopka et al., Proc. Natl. Acad. Sci. U.S.A. 82, 1810 (1985)). The 210 kDa protein is considerably larger than the normal human abl protein of 145 kDa, and has a very high tyrosine kinase activity.

Two major types of bcr-abl translocations are known, characterized by two different bcr-abl junctions. One translocation is between bcr exon 2 and abl exon 2, while another translocation is between bcr exon 3 and the same abl exon 2 (Shtivelman et al., Cell 47, 277-284 (1986)). The two types of junction have been referred to as the "L-6" (or "b2a2") and "K-28" (or "b3a2") junctions, respectively. The alternative splicing from two bcr-abl exons to the abl coding sequence results in two different bcr-abl fusion proteins, one including the 25 amino acids encoded by bcr exon 3 and one which lacks those amino acids. One or both of these junctions is detected in Ph¹-positive CML patients (Shtivelman et al., Blood 69, 971 (1986)).

A significant portion of acute lymphocytic leukemia (ALL) patients carry Ph¹ chromosomes in their leukemic cells. Ph¹-positive ALL is generally regarded as being less responsive to chemotherapeutic treatment than Ph¹-negative forms of ALL. This is particularly true of children with Ph¹-positive ALL.

Approximately one half of Ph¹-positive individuals afflicted with ALL express either of the two major bcr-abl junctions, L-6 or K-28. The remainder have bcr-abl genes characterized by a junction formed by the fusion of bcr exon 1 and c-abl exon 2 ("bla2" junction). See Fainstein et al., Nature 330, 386-388 (1987).

There are thus at least three distinct bcr-abl mRNAs. The three mRNAs contain one of three different bcr exons fused to the same abl exon. About one half of CML patients have the b2a2 junction, while the other half are characterized by the b3a2 junction. ALL patients are about fifty percent b1a2, twenty-five percent b2a2 and twenty-five percent b3a2. An improved polymerase chain reaction (PCR) procedure has been proposed for distinguishing among the three types of molecular defects using analyses of PCR reaction products by hybridization with probes specific for the three known bcr-abl fusion sequences (Kawasaki et al., Prod. Anal. Acad. Sci. USA 85, 5698-5702 (1988)).

Clinically, CML invariably progresses from the chronic phase into the blast crisis. In chronic phase CML, the increase in mature and immature myeloid elements in bone marrow and peripheral blood is the most characteristic feature (Koeffler et al., N. Engl. J. Med. 304, 201 (1981)). Kinetic studies indicate that these abnormal cells do not proliferate or mature faster than their normal counterparts. Instead, the basic defect underlying the exuberant granulopoiesis in CML appears to reside in the expansion of the myeloid progenitor cell pool in bone marrow and peripheral blood. Id. Nevertheless, the generation of terminally differentiated cells indicates that the process of hematopoiesis retains some normal features. In contrast, during blastic transformation, the leukemic cells exhibit a marked degree of differentiation arrest with a "blast" phenotype (Rosenthal et al., Am. J. Med. 63, 542 (1977)). The onset of the blastic transformation or "blast crisis" limits the therapeutic options available. The disease-free period, and consequently survival, is generally brief. Typically it is less than about four months.

According to a preferred embodiment of the practice of the present invention, Ph¹-positive leukemias are treated, either in vivo or ex vivo, with a combination of antisense oligonucleotide specific for bcr-abl mRNA and one or more non-antisense chemotherapeutic agents, including but not limited to 4-HC, mafosfamide, etoposide, cisplatin, vincristine, prednisone, L-asparaginase, methotrexate, and combinations thereof.

Preferably, the bcr-abl antisense oligonucleotide is complementary to a position of the bcr-ablmRNA corresponding to the breakpoint junction between the bcr-derived and abl-derived portions of the mRNA. By "abl-derived portion" is meant that portion of the bcr-abl RNA transcript which results from the transcription of the abl coding sequence which is translocated to the bcr coding sequence in the chromosomal translocation event giving rise to formation of the Ph¹ chromosome. Similarly, by "bcr-derived portion" of the bcr-abl transcript is meant that portion which results from the transcription of the bcr coding sequence which is juxtaposed to c-abl. This ensures specific hybridization to bcr-abl transcripts. To further ensure specific hybridization of the therapeutic antisense oligonucleotide to bcr-abl transcripts, it is preferred that the oligonucleotide has a sequence including from about 6 to about 13 abl-derived nucleotides, the balance of the antisense oligonucleotide being complementary to bcr-derived nucleotides of the target sequence. Most preferably, the antisense molecule is complementary to a target mRNA sequence containing an about equal number of abl-derived nucleotides and bcr-derived nucleotides, that is, an about equal number of nucleotides on either side flanking the translocation breakpoint. Accordingly, one group of most preferred antisense oligonucleotides complementary to the b2a2 junction includes the following even-numbered 14 through 26-mers:

Correspondingly, the following even-numbered 14- through 26-mers comprise most preferred antisense oligonucleotides complementary to, respectively, the b3a2 junction, and the bla2 junction:

The initial step in the therapeutic method of the invention is the identification of the patient-to-be-treated as possessing the hybrid bcr-abl gene. This may be accomplished by probing the patient RNA or cDNA with suitably labeled nucleic acid probes for bcr-abl, such as those disclosed in U.S. patents 4,681,840 and 4,874,853, the entire disclosures of which are incorporated herein by reference. Total RNA may be probed with one or more of the above-listed antisense oligonucleotides. Finally, molecular diagnosis of Ph¹-positive leukemias could be achieved by amplification and detection of characteristic mRNA sequences utilizing a reverse transcriptase polymerase chain reaction (RT-PCR) procedure, such as the procedure disclosed by Kawasaki et al., Proc. Natl. Acad. Sci. USA, 85, 5698-5702 (1988), incorporated herein by reference.

Upon diagnosis establishing the presence of the bcr-abl gene, leukemic Ph¹-positive cells are obtained from the peripheral blood and/or bone marrow of the patient for sequencing of the bcr-abl junction. The cells may be enriched by procedures such as Ficoll-Hipaque centrifugation to remove non-mononuclear cells. RNA containing the nucleotide sequence corresponding to the bcr-abl hybrid gene is extracted for reverse transcription, amplification and sequencing. Preferably, the source of bcr-abl nucleotide sequence information comprises RNA.

Accordingly, total mRNA is isolated from the Ph¹-positive enriched cells according to well-known extraction procedures, such as the procedures described in Molecular Cloning: A Laboratory Manual (2d. ed. 1989), J. Sambrook et al, eds., pp. 7.9-7.11, incorporated herein by reference. In particular, a single step RNA isolation method may be utilized, such as the acid guanidinium thiocyanate-phenol-chloroform extraction method described by Chomzcynski et al., Anal. Biochem. 162, 156-159 (1987), incorporated herein by reference. The bcr-abl junction is thereafter cloned by any of the known amplification techniques, most preferably by RT-PCR. Accordingly, synthetic primers specific for bcr exon 2 and abl exon 2 are utilized in a RT-PCR technique to clone the b2a2 junction. Similarly, synthetic primers specific for bcr exon 3 and abl exon 2 are utilized for amplifying the b3a2 junction. Such synthetic primers may be prepared based upon the published sequences for the b2a2 and b3a2 breakpoint junctions (Shtivelman et al., Cell 47, 277-286 (1986), incorporated herein by reference, and Fainstein et al., Nature 330, 386-388 (1987), incorporated herein by reference).

Following the amplification step, the polymerase chain reaction product may be sequenced directly. Alternatively, the product may be further amplified by cloning in a suitable vector, e.g., the BLUESCRIPT SK (M13-) vector (Stratagene Cloning Systems, La Jolla, CA), which is described in Molecular Cloning, p. 1.20 and Short et al., Nucleic Acids Res. 16, 7583 (1988). Sequencing of the relevant region around the bcr-abl breakpoint of the cloned polymerase chain reaction product is then carried out according to conventional sequencing procedures, such as described in Molecular Cloning, chapter 13, incorporated by reference.

Antisense oligonucleotides having a sequence complementary to the relevant bcr-abl breakpoint of the individual patient are then prepared, based upon the sequence information obtained. In general, the antisense oligonucleotide will have a sequence which is completely complementary to the target sequence of the bcr-abl message.

The bcr-abl antisense oligonucleotide and non-antisense agent are then used to treat the aspirated bone marrow of the Ph¹-positive patient. In addition to their use in treating the patient's aspirated bone marrow, one or both of the agents may also be administered to the patient directly, such as by intravenous injection or infusion, either before or after the harvesting of bone marrow cells.

The practice of the invention is illustrated by the non-limiting examples, below.

### Comparative Example 1

### Effect of Mafosfamide on In Vitro Colony-Forming Ability of Normal and CML Marrow Cells

### A. Normal and Primary Leukemic A⁻T⁻MNC Cells

Marrows were obtained as iliac crest aspirates from 8 healthy volunteers after informed consent. Light-density mononuclear cells (MNC), separated on Histopaque-1077 (Sigma, St. Louis, MO) density gradient were enriched for hematopoietic progenitors by removal of adherent cells as described by Skorski et al., J. Exp. Med. 175, 743-750 (1992). MNC from 11 chronic myelogeneous leukemia patients in blast crisis (CML-BC) and 7 patients in the chronic phase of the disease (CML-CP) were similarly isolated from bone marrow.

### B. Mafosfamide Treatment

Mafosfamide was obtained from Asta-Werke AG, Germany. It was dissolved in Iscove's modified Dulbecco medium (IMDM) and sterilized by filtration through a 0.22 *µ*m Acrodisc filter (Gelman Sciences, Ann Arbor, MI) before use. A⁻T⁻MNC, CML-BC or CML-CP cells (10⁶-10⁷ cells/ml) in IMDM supplemented with 10% heat-inactivated human AB serum were incubated in the presence of different concentrations of mafosfamide for 30 minutes at 37°C, washed and resuspended in IMDM medium.

### C. Colony Assay

5 x 10⁴ of mafosfamide-treated A⁻T⁻MNC, CML-BC or CML-CP cells were plated in methylcellulose (HCC-4230 medium, Terry Fox Laboratory, Vancouver, CN) supplemented with IL-3 (20 U/ml), GM-CSF (5 ng/ml), and 0.125 mM L-glutamine in duplicate 35-mm Petri dishes (Nunc Inc., Naperville, IL) at 1 ml/dish. Colonies and clusters were scored after 9-12 days of culture in a humidified 5% CO₂ incubator. The results are shown in Figs. 1A-1D for the following concentrations of mafosfamide: 1A, 12.5 *µ*g/ml; 1B, 25 *µ*g/ml; 1C, 50 *µ*g/ml; and 1D, 100 *µ*g/ml. Error bars represent mean values ± the standard deviation. (Hollow bar, A⁻T⁻MNC; solid bar, CML-BC; cross-hatched bar, CML-CP). The leukemia cells were more sensitive than normal hematopoietic cells to mafosfamide at concentrations of 25, 50 and 100 *µ*g/ml. At the highest concentration of mafosfamide (100 *µ*g/ml), formation of colonies derived from CML-BC or CML-CP cells was completely inhibited in samples from 9/11 patients and 6/7 patients, respectively, while only 3.15% (0.6-10.3%) of normal hematopoietic colonies were spared in 8/8 A⁻T⁻MNC samples from healthy volunteers. The CD34+ subpopulation of CML cells was also more sensitive than that of normal hematopoietic progenitors to mafosfamide (data not shown). Secondary colony formation assays to further demonstrate that mafosfamide treatment at 100 *µ*g/ml permanently inhibited the growth ability of leukemic cells but not of normal progenitors indicated no visible secondary colonies derived from CML primary cells, but indicated several CFU-GM colonies from normal A⁻T⁻MNC incubated with the same concentrations of the drug.

### Comparative Example 2

### Effect of Mafosfamide on Colony Formation and bcr-abl Expression in 1:1 Mix of CML-BC Primary Cells and Normal Marrow Cells

### A. Colony Assay

To determine whether mafosfamide preferentially eliminates leukemic cells as compared to normal progenitor cells, normal A⁻T⁻MNC and CML-BC cells mixed at a 1:1 ratio were incubated with increasing concentrations of mafosfamide and plated in methylcellulose, and the resulting colonies were counted, as in Example 1. The results are shown in Fig. 2 (graph).

### B. RNA Extraction and RT-PCR

The cells were then harvested, centrifuged on Histopaque-1077 to eliminate dead cells and washed, followed by total RNA extraction in the presence of 20 *µ*g of E. coli ribosomal RNA as described by Chomczynski et al., Anal. Biochem. 162, 156-159 (1987). RNA from each group was divided into two aliquots. One sample was reverse-transcribed using 400 U of Moloney murine leukemia virus reverse transcriptase (BRL, Gaithersburg, MD) and 0.1 *µ*g of 3 primer of abl exon 2 for 1 hour at 37°C. The second sample was reverse-transcribed using the β₂-microglobulin 3 primer. The resulting DNA fragments were amplified with 5 U of Thermus aquaticus (Taq) polymerase (Perkin Elmer Cetus, Norwalk, CT) in the presence of the 5 primer of bcr exon 3 or β₂-microglobulin 5 primer, generating a 266-bp fragment corresponding to the bcr-abl breakpoint region, and a 252-bp fragment of β₂-microglobulin, during 56 cycles of PCR (Saiki et al., Science 239, 487-491 (1984)). Reaction products were electrophoresed in 2% agarose gel, transferred to Zeta-probe blotting membranes (Bio-Rad, Richmond, CA) and hybridized overnight at 50°C using the abl exon 2 or β₂-microglobulin probes end-labeled with [γ⁻³²]P-ATP and polynucleotide kinase as described in Molecular Cloning: A Laboratory Manual, Maniatis et al. eds., Cold Spring Harbor, New York: Cold Spring Harbor Laboratory, 1982. Filters were washed with 2X SSC + 0.1% SDS at 49°C for 30 minutes. No bcr-abl mRNA was detected after treatment with mafosfamide at 100 *µ*g/ml, and the bcr-abl transcript was barely detectable after exposure to mafosfamide at 50 *µ*g/ml (Fig. 2, bottom). By contrast, control *β*₂-microglobulin mRNA was clearly detectable in all samples (Fig. 2, bottom). These representative results obtained with cells from a CML-BC patient indicate that cells carrying the Ph¹ translocation and generating bcr-abl transcript were completely eliminated, whereas normal progenitor cells survived exposure to mafosfamide at 100 *µ*g/ml.

### Example 3

### Effect of Mafosfamide and/or bcr-abl Antisense Oligodeoxynucleotide on Colony Formation and bcr-abl Expression in 1:1 Mix of BV173 and Normal Marrow Cells

The combination of mafosfamide and bcr-abl antisense oligodeoxynucleotide was tested for leukemic cell purging activity at concentrations which, when used separately, did not eliminate totally the leukemic cells but spared 47 and 85% of hematopoietic clonogenic cells respectively.

### A. BV173 Cells

The Ph¹ leukemia cell line BV173 was established from a patient in lymphoid blast crisis (Pegoraro et al., J. Natl. Canc. Inst. 70, 447-450 (1983)). It is characterized by the presence of the b2a2 breakpoint in which the bcr exon 2 is fused to the abl exon 2 (van Denderen et al., Blood 76, 136-141 (1990). The cell line is also characterized by the presence of the common acute lymphoblastic leukemia antigen (CALLA), and the absence of CD45 antigen (Id.). BV173 cells form colonies with high efficiency when plated in semisolid medium. Proliferation is inhibited by exposure to mafosfamide. Proliferation is inhibited by exposure to a b2a2 antisense 18-mer (SEQ ID NO:1) complementary to the 18 nucleotides of the b2a2 mRNA corresponding to the nine nucleotides upstream and downstream of the breakpoint junction. Proliferation is not inhibited by the corresponding b2a2 sense oligomer (SEQ ID NO:2), or b1a2 or b3a2 antisense oligomers (data not shown).

### B. Oligomer Treatment

A 1:1 mixture of BV173 and normal A⁻T⁻MNC cells (0.5-1.0 x 10⁶ cells/ml were seeded into 24 well cell culture plates (Cosdar Corp., Cambridge, MA) in 0.4 ml IMDM supplemented with 2% heat-inactivated human AB serum, Hepes buffer and recombinant human IL-3 (50 U/ml) and GM-CSF (12.5 ng/ml) (Genetics Institute, Cambridge, MA). The cells were incubated with mafosfamide (2.5 *µ*g/ml) and/or b2a2 bcr-abl antisense oligodeoxynucleotide (SEQ ID NO:1) or the corresponding sense oligodeoxynucleotide (SEQ ID NO:2) (80 *µ*g/ml at time zero, followed by a second dose of 40 *µ*g/ml at 18 hours, and a third dose of 40 *µ*g/ml at 40 hours).

### C. Colony Assay

Colony formation assays performed in accordance with the procedure of Example 1 revealed residual colonies in the presence of 2.5 *µ*g/ml mafosfamide or b2a2 antisense oligodeoxynucleotides (80+40+40 *µ*g/ml), and also when mafosfamide (2.5 *µ*g/ml) was combined with the b2a2 antisense oligomer (80+40 *µ*g/ml). See Fig. 3 (graph). (Error bars represent the mean value plus the standard deviation).

### D. RNA Extraction and RT-PCR

RNA extraction and RNA phenotyping by RT-PCR in accordance with the procedure of Example 2 revealed that a bcr-abl transcript was clearly present in colonies derived from cells treated with mafosfamide alone or with antisense oligomer alone, but was not detectable after exposure to a combination of the two reagents, indicating that mafosfamide and bcr-abl antisense oligonucleotide have a synergistic effect against leukemic cells (Fig. 3, bottom). The combination of mafosfamide and bcr-abl antisense oligodeoxynucleotide purged all of the leukemic cells from normal bone marrow cells as indicated by the disappearance of the bcr-abl transcript. In contrast, control β₂-microglobulin mRNA was clearly detectable in each sample (Fig. 3, bottom), suggesting that normal clonogenic cells were spared.

### E. Cytogenic Analysis

A 1:1 mix of normal marrow progenitors and leukemic cells (BV173 or CML-BC) was cultured in methylcellulose in the presence of bcr-abl antisense oligodeoxynucleotide (SEQ ID NO:1), alone or in combination with mafosfamide. After 7-9 days, plates were exposed to a colcemide solution (0.3 *µ*g/ml) for 1 hour. Single colonies were plucked from the methylcellulose, centrifuged and washed three times with physiological saline to remove residual methylcellulose. After 25 minutes of hypotonic treatment in 0.057 M KCl, cells were fixed by washing several times with methanol: glacial acetic acid (3:1). Slides were air-dryed and G-banded as described by Seabright, Lancet 2, 971-972 (1971).

Cytogenic analysis of the individual colonies derived from the 1:1 mixture of normal A⁻T⁻MNC and BV173 cells, untreated or exposed to a combination of bcr-abl antisense oligodeoxynucleotide and mafosfamide, revealed that 15 of 20 untreated colonies were Ph¹-positive, whereas all 18 colonies examined after treatment with bcr-abl antisense oligodeoxynucleotides and mafosfamide, were karyotypically normal. See Table 1. (A 1:1 mixture of CML-BC and A⁻T⁻MNC mixed cells were treated in the same fashion as the BV173/A⁻T⁻MNC mixture.) The complete absence of Ph¹-positive metaphases excludes the possibility of the growth of "silent" leukemic cells that possess the Ph¹ chromosome but do not express bcr-abl transcripts (Keating et al.), Exp. Hematol. 19, 539 (1991), and confirms the high efficiency of the purging procedure.

**TABLE 1**

| Cytogenic Analysis for Ph¹ Chromosome in Colony Forming Cells Before or After Purging | | |
|---|---|---|
| Cells^{a} | No. of Ph¹ Colonies per No. of Colonies Analyzed | |
| | untreated | treated |
| BV173 + A⁻T⁻MNC | 15/20 | 0/18 |
| CML-BC + A⁻T⁻MNC | 12/18 | 0/23 |

| | | |
|---|---|---|
| ^{a} 1:1 mixture of leukemic and normal cells. | | |

Methaphases of BV173 and CML-BC patient cells were 100% positive for Ph¹ before the studies. Bcr-abl sense oligodeoxynucleotides did not exert any additional effect against leukemic cells when combined with mafosfamide (data not shown).

### Example 4

### Detection of Normal Hematopoietic Colony-Forming Cells But not Leukemic Cells in SCID Mice Injected with a 1:1 Mixture A⁻T⁻MNC and BV173 Cells Treated with Mafosfamide and bcr-abl Oligodeoxynucleotide.

The following in vivo study confirms the in vitro results obtained in the preceding example. Human bone marrow cells mixed with BV173 leukemic cells were treated with mafosfamide and bcr-abl antisense oligodeoxynucleotide before injection into immunodeficient SCID mice supported with human recombinant hematopoietic growth factors (Lapidot et al., Science 255, 1137-1141 (1991)). Assays for the presence of human α-satellite DNA sequence and expression of bcr-abl mRNA in single colonies growing in vitro from bone marrow cells of SCID mice revealed only normal human hematopoiesis after implantation of purged bone marrow, and mostly leukemic hematopoiesis after injection of nonpurged bone marrow.

### A. Mice

Triple immunodeficient bg/nu/xid (BNX) female mice and immunodeficient SCID male mice were obtained from Taconic (Germantown, N.Y.). Mice were maintained under pathogen-free conditions and were 6-8 weeks old when used in the following experiments. BNX and SCID mice received 600 cGy and 300 cGy of total body irradiation, respectively, one day before injection of normal or leukemic cells as hereinafter described.

### B. Injection of Normal or Leukemic Cells

Cells (human A⁻T⁻MNC or BV173) were seeded into a 75-cm² Corning tissue culture (Corning Glass Works, Corning, N.Y.), treated with 2.5 *µ*g/ml mafosfamide, and then incubated with sense (SEQ ID NO:2) or antisense (SEQ ID NO:1) b2a2 oligodeoxynucleotide at time zero, followed by a second dose of 40 *µ*g/ml at 18 hours, and a third dose of 40 *µ*g/ml at 40 hours. A 1:1 mixture of the A⁻T⁻MNC and BV173 cells, untreated or treated with mafosfamide and the b2a2 antisense oligonucleotide, was washed with IMDM and injected into the SCID mice. Following injection of the cells, the mice were supported with stem cell growth factor (SCF, 20 *µ*g), a fusion protein of human interleukin-3 (IL-3) and granulocyte-macrophage colony stimulating factor (GM-CSF) (PIXY 321, 8 *µ*g), and erythropoietin (EPO, 20 U). Mice were injected intraperitoneally with the growth factors every two days for a total of 15 times. SCF and PIXY 321 were obtained from Immunex Corp. (Seattle, WA). EPO was obtained from Amgen, Inc. (Thousand Oaks, CA).

### C. Flow Cytometry Analysis

The mice were sacrificed one month after injection. Single cell suspensions were prepared from bone marrow of the mice injected with human cells. Cells (10⁵) were stained with FITC-conjugated mouse anti-HLe-1 (anti-CD45) or anti-CALLA (anti-CD10) monoclonal antibody (Becton-Dickinson Immunocytometry Systems, San Jose, CA), washed and analyzed by flow cytometry using the Epics Profile Analyzer (Coulter Electronics). For each cell type, two negative controls were used: the same cell population stained with FITC-conjugated anti-human CD3 monoclonal antibody, and bone marrow cells stained with FITC-conjugated anti-HLe-1 or anti-CALLA from noninjected mice. The analysis revealed CD45+ cells in all SCID mice injected with purged and nonpurged human bone marrow (Table 2),

**TABLE 2**

| Effect of Mafosfamide Plus bcr-abl Antisense Oligodeoxynucleotide on the Number of Leukemic and Normal Hematopoietic Colony-forming Cells in SCID Mice Bone Marrow; Mice Injected with 1:1 A⁻T⁻MNC/BV173 Mixture | | | | |
|---|---|---|---|---|
| Treatment^{a} | cells (x10⁶)^{b} | %CD45+ cells | % CALLA+ cells | colonies /10⁵ cells |
| None | 22.5 23.0 | 7.0 ± 0.3 | 15.3 ± 7.3 | 3121.0 ± 210.7 |
| MAF + antisense | 22.5 6.0 | 3.2 ± 1.2 | 0 | 34.5 ± 21.9 |

| | | | | |
|---|---|---|---|---|
| ^{a} Two mice per group were analyzed. The results represent mean ± SD from six determinations. | | | | |
| ^{b} Left column: number of human cells before treatment; right column: number of cells injected in the mouse after treatment. | | | | |

indicating the presence of normal hematopoietic cells (BV173 leukemia cells do not express this antigen). CALLA+ cells (mainly BV173 cells) were detected only in mice injected with nonpurged bone marrow. Under conditions which presumably allow only the growth of human cells (bone marrow progenitors and BV173 cells), several colonies (35.0 ± 22/10⁵ cells plated) were detected in bone marrow of mice injected with the 1:1 mixture of normal marrow cells and BV173 cells exposed to mafosfamide (2.5 *µ*g/ml) and b2a2 antisense oligodeoxynucleotides (80+40+40 *µ*g/ml) (Table 2).

### D. Single Colony PCR Analysis

These colonies (10 colonies tested) were of human origin, as indicated by the detection of human-specific α-satellite DNA sequences, and were not leukemic because of the lack of bcr-abl mRNA expression. The colonies showed a characteristic myeloid or erythroid phenotype. Numerous colonies (3121.0 ± 210.7/10⁵ cells plated) arose from bone marrow of SCID mice injected with nonpurged cells (Fig. 4). The human α-satellite DNA sequence and bcr-abl mRNA were easily detectable in single colonies (10 colonies tested), indicating the leukemic origin of these colonies (Fig. 4). The phenotype of the colonies was typical for colonies derived from BV173 cells.

### Example 5

### Effect of Mafosfamide Plus bcr-abl Antisense Oligodeoxynucleotides on CML-BC or A⁻T⁻MNC Cells Colony Formation

Clonogenic assays of CML-BC cells from 4 patients who were either the b2a2 or b3a2 phenotype and A⁻T⁻MNC cells from 3 healthy volunteers were utilized to determine the sensitivity of these cells to a combination of mafosfamide (25 *µ*g/ml) and bcr-abl antisense oligodeoxynucleotides (80+40+40 *µ*g/ml). Cells were incubated with the concentrations of mafosfamide indicated in Table 3 and left untreated (C) or treated with breakpoint-specific sense (S) or antisense (AS) oligodeoxynucleotides. (b3/a2 antisense = SEQ ID NO:3; b3/a2 sense = SEQ ID NO:4) The results shown in Table 3 (mean ± SD) are from duplicate cultures. ND=not determined.

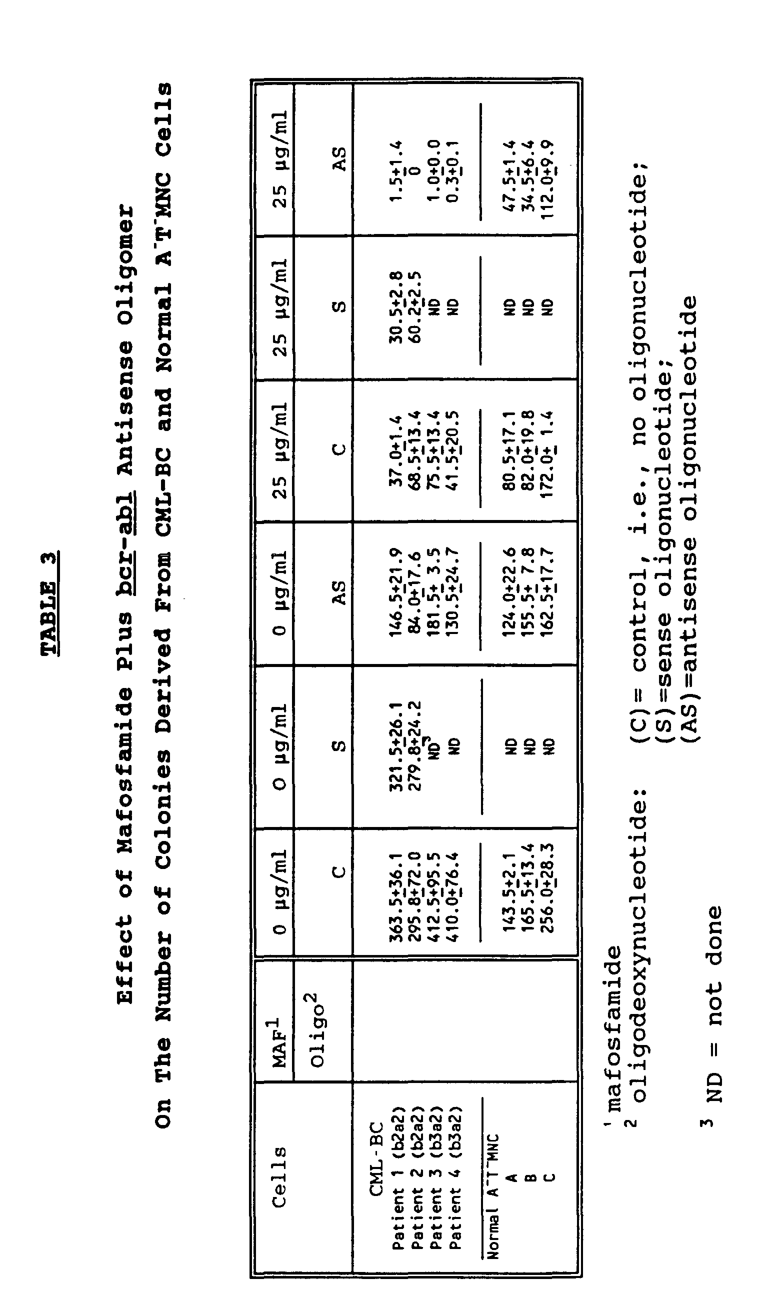

Compared to untreated cultures, CML-BC colony formation was completely suppressed by exposure to mafosfamide plus antisense oligodeoxynucleotides (99.8-100% inhibition). Under identical culture conditions 20.8-43.8% of normal hematopoietic progenitors were spared (Table 3). At those concentrations, mafosfamide or antisense oligodeoxynucleotides alone were not able to inhibit completely the growth of CML-BC cells.

### Example 6

### Effect of Mafosfamide Plus bcr-abl antisense Oligodeoxynucleotides on a 1:1 Mixture of CML-BC and Normal Marrow Cells.

Primary cells from a CML-BC patient in whom cytogenetic analysis indicated that 100% metaphases were Ph¹-positive and molecular analysis revealed the expression of bcr-abl transcripts containing the b2a2 breakpoint junction were mixed with an equal number of A⁻T⁻MNC and incubated with mafosfamide (25 *µ*g/ml) and bcr-abl antisense oligomer (SEQ ID NO:1) complementary to the breakpoint junction (b2a2, 80+40+40 *µ*g/ml) before plating in methylcellulose. Colonies were counted after 9 days of culture, harvested from the plates and washed to remove residual methylcellulose before isolation of total RNA (Fig. 5, graph). A bcr-abl transcript was clearly present in untreated cells and in cells treated with mafosfamide or with the antisense oligomer alone, but was not detectable after exposure to a combination of mafosfamide and bcr-abl antisense oligodeoxynucleotide (Fig. 5, bottom). In contrast, control β₂-microglobulin mRNA was clearly detectable in each sample (Fig. 5, bottom). Bcr-abl sense oligodeoxynucleotides in combination with mafosfamide had no additional effect over that of mafosfamide alone (data not shown). Cytogenetic analysis of individual colonies derived from a 1:1 mix of normal marrow mononuclear cells and leukemic cells exposed to a combination of bcr-abl antisense oligodeoxynucleotide and mafosfamide revealed a normal karyotype in all 23 colonies examined, whereas only 6 of 18 untreated colonies were normal and the remaining 12 were Ph¹-positive (Table 1). Together, these results indicate that only the combination of mafosfamide and bcr-abl antisense oligodeoxynucleotide eliminated cells carrying the Ph¹ translocation and spared a high number of normal progenitor cells.

### Example 7

### Engraftment of Human Normal but not Patient Leukemic Hematopoietic Cells in SCID Mice Injected with a 1:1 Mixture of Marrow Mononuclear Cells and CML-BC Primary Cells After Mafosfamide and BCR-ABL Antisense Oligonucleotides Treatment

The following in vivo study confirms the in vitro results obtained in the preceding Example. Human bone marrow cells mixed with CML-BC primary cells were treated with mafosfamide bcr-abl antisense oligonucleotide before injection into immunodeficient SCID mice supported with human recombinant hematopoietic growth factors. This murine model allowed detection of normal and primary CML-BC cells in host mice after injection of at least 10⁶ cells. Assays for the presence of human α-satellite DNA sequence and expression of bcr-abl mRNA in single colonies growing in vitro from bone marrow cells of SCID mice revealed only normal human hematopoietis after implantation of purged bone marrow, and mostly leukemic hematopoiesis after injection of non-purged bone marrow.

A 1:1 mixture of A⁻T⁻MNC and CML-BC patient cells (60 x 10⁶; b2a2 breakpoint) were divided into two portions and were treated with mafosfamide (25 *µ*g/ml) plus b2a2 antisense oligodeoxynucleotide (SEQ ID NO:1; 80+40+40 *µ*g/ml) or left untreated. Severe combined immunodeficient (SCID) male mice, 6-8 weeks of age (Tac-onic, Germantown, NY) received 300 cGy of total body irradiation. One day following irradiation, the mice were intravenously injected with the treated cells. The mice were then supported with SCF (20 *µ*g) PIXY 321 (8 *µ*g) and EPO (20 U). The mice were injected intraperitoneally with the growth factors every two days for a total of fifteen times.

One month after injection of cells, the mice were sacrificed and bone marrow cells were isolated. Flow cytometric analysis was conducted according to the procedure of Example 4. The analysis revealed the presence of CD45+ cells in both groups of mice (Table 4):

**TABLE 4**

| Effect of Mafosfamide Plus bcr-abl Antisense Oligodeoxynucleotide on the Number of Leukemic and Normal Hematopoietic Colony-forming Cells in SCID Mice Bone Marrow; Mice Injected With 1:1 A⁻T⁻MNC/CML-BC Mixture | | |
|---|---|---|
| Treatment^{a} | %CD45+ cells | Colonies /10⁵ cells |
| None | 24.0±3.6 | 149.8 ± 18.3 |
| MAF + antisense | 1.5±0.2 | 9.8 ± 4.0 |

| | | |
|---|---|---|
| ^{a} Two mice per group were analyzed. The results represent mean ± SD from six determinations. | | |

Under conditions that favor the growth of human hematopoietic cells, numerous colonies were grown from bone marrow of mice injected with nontreated cells, whereas only few colonies grew after injection of purged cells (Table 4).

Single colony PCR analysis was conducted according to the procedure of Example 4. All colonies (5 colonies tested/mouse) contained human-specific α-satellite DNA sequences (Fig. 12). In contrast, bcr-abl mRNA was only detected in four colonies which arose from bone marrow of the mouse injected with nontreated cells (5 colonies tested/mouse) (Fig. 12), not from mice injected with purged cells. The analysis of single colonies from the second group of mice gave similar results.

### Example 8

### Engraftment of Human Hematopoietic Colony-forming Cells Treated with Mafosfamide and bcr-abl Antisense Oligodeoxynucleotides in BNX Mice

To even more closely mimic a clinical context, immunodeficient BNX mice were injected in the following experiment with human bone marrow cells incubated under conditions that allow the in vitro elimination of primary CML-BC cells, i.e., mafosfamide (100 *µ*g/ml) or mafosfamide (25 *µ* g/ml) plus bcr-abl antisense oligodeoxynucleotides (80 *µ*g/ml at time zero; 40 *µ*g/ml at 18 hours; 40 *µ*g/ml at 40 hours).

A⁻T⁻MNC were treated with mafosfamide alone (100 *µ*g/ml), or with mafosfamide (25 *µ*g/ml) plus b2a2 antisense oligodeoxynucleotide (80+40+40 *µ*g/ml) as described above, and assayed for colony-forming ability in vitro in the presence of rhu IL-3 and rhu GM-CSF before injection into sublethally irradiated BNX mice. Untreated cells formed numerous colonies (707 ± 80 colonies per 10⁵ cells), whereas colony formation was diminished (183 ± 50 colonies per 10⁵ cells) after mafosfamide plus bcr-abl antisense oligodeoxynucleotide treatment, and almost totally inhibited (3 ± 3 colonies per 10⁵ cells), after treatment with mafosfamide (100 *µ*g/ml) alone.

One or two months after cell injection, the mice were sacrificed, bone marrow cells were isolated from sacrificed mice and examined for the presence of human hematopoietic colony-forming cells by monoclonal antibody staining and colony-forming ability. The results are set forth in Table 5. Experiments "A" and "B" represent analyses one month after cell injection. Experiment "C" corresponds to an analysis two months post cell injection. Human hematopoietic clonogenic cells (CD45+) cells were detected among bone marrow cells obtained from mice injected with untreated- or mafosfamide (25 *µ*g/ml) plus b2a2 antisense oligodeoxynucleotide-treated human bone marrow cells, but not from mice injected with mafosfamide-treated (100 *µ*g/ml) cells.

**TABLE 5**

| Engraftment of Human Hematopoietic Colony Forming Cells in BNX Mice | | | | |
|---|---|---|---|---|
| Mice^{a} | A⁻T⁻MNC injected (10⁶/mouse)^{b} | | CD45+ cells /10⁵ cells | colonies/10⁵ cells^{c} |
| CONTROL | | | | |
| A | 3.3 | 1.5 | 349±70 | 5,12,4,2 |
| B | 2.3 | 2.5 | 140±8 | 4,4,8,6 |
| C | 4.0 | 2.8 | 355±177 | 3,0,1,4 |
| | | | | |

| MAF/100 | | | | |
|---|---|---|---|---|
| A | 3.3 | 0.7 | 0 | 0 |
| B | 2.3 | 1.1 | 0 | 0 |
| C | 4.0 | 1.6 | 0 | 0 |
| | | | | |

| MAF/100 +AS | | | | |
|---|---|---|---|---|
| A | 3.3 | 1.0 | 398±98 | 7,11,3,1 |
| B | 2.3 | 1.9 | 60±11 | 8,4,4,10 |
| C | 4.0 | 2.2 | 335±35 | 1,1,0,3 |

| | | | | |
|---|---|---|---|---|
| ^{a} Mice were analyzed one (experiments A and B) or two months (C) after implantation of human bone marrow cells. | | | | |
| ^{b} Left column: numbers of human A⁻T⁻NBMC in each experiment (A,B,C) before treatment; right column: numbers of the cells injected into the mouse after treatment | | | | |
| ^{c} Number of colonies/plate | | | | |

Under conditions that allow only growth of human bone marrow progenitor cells, several myeloid and erythroid colonies were detected in bone marrow of mice injected with untreated bone marrow cells or mafosfamide (25 *µ*g/ml) plus b2a2 antisense oligodeoxynucleotide-treated human bone marrow cells (2-12 or 1-11 per 10⁵ cells, respectively), but not in bone marrow of mice injected with mafosfamide-treated (100 *µ*g/ml) cells (Table 5). The human origin of most of these colonies (5/6) was confirmed by detection of human α-satellite DNA sequence in PCR reaction products obtained from single colonies (Fig. 6). DNA from human (H) and murine (M) peripheral blood mononuclear cells served as positive and negative controls, respectively.

These experiments provide strong evidence that numerous immature human hematopoietic cells, some of which persist in immunodeficient mice, are spared after mafosfamide plus antisense oligodeoxynucleotide treatment but not after mafosfamide (100 *µ*g/ml) treatment.

### Example 9

### Effect of bcr-abl Antisense Oligonucleotide and Adriamycin On BV173 Cells

BV173 cells (10⁷ cells/ml of RPMI + FBS) were incubated in vitro at 37°C for 60 min. with adriamycin (doxorubicin HC1, Adria Labs, Columbus, OH). The cells were then washed and incubated in RPMI plus 10% FBS (10⁶ cells/ml) with b2a2 sense (SEQ ID NO:2) or antisense (SEQ ID NO:1) oligodeoxynucleotides at a concentration of 80 *µ*g/ml for the first 18 hours. A second dose of 40 *µ*g/ml oligonucleotide was added and the cells were incubated for the next 24 hours. After incubation was completed, 2.5-10 x 10³ cells were plated in methylcellulose medium (HCC-4230, Terry Fox Lab., Vancouver, CN) in duplicate 35-mm Petri dishes (Nunc Inc.). The colonies were counted after 7-9 days. The results, set forth in Fig. 7A (12.5 *µ*g/ml adriamycin) and 7B (25 *µ*g/ml adriamycin), represent the mean ± the standard deviation of 2-4 independent experiments.

### Example 10

### Effect of bcr-abl Antisense Oligonucleotide and Etoposide On BV173 Cells

The procedure of Example 9 was repeated, substituting etoposide (Bristol Labs, Evansville, IN) (37°C incubation for 30 min.) for adriamycin. The results, set forth in Fig. 8A (3.125 *µ* g/ml etoposide) and 8B (6.25 *µ*g/ml etoposide), represent the mean ± the standard deviation of 2-4 independent experiments.

### Example 11

### Effect of bcr-abl Antisense Oligonucleotide and Platinol on BV173 Cells

The procedure of Example 9 was repeated, substituting platinol (cisplatin, Bristol Labs, Evansville, IN) (37°C incubation for 60 min.) for adriamycin. The results, set forth in Fig. 9A (12.5 *µ*M/ml platinol) and 9B (25 *µ*M/ml platinol), represent the mean ± the standard deviation of 2-4 independent experiments.

### Example 12

### Effect of c-myb Antisense Oligonucleotide and Adriamycin on LAN-5 Cells

LAN-5 cells, which comprise a human neuroblastoma cell line, were seeded in a 24-well plate (Costar Corp., Cambridge, MA) in a concentration of 10⁴ cells/ml of RPMI plus 15% FBS. After overnight incubation, which allows the cells to attach to the bottom of the plate, 0.5 or 1 *µ*g/ml adriamycin were added and the cells incubated for 60 min. at 37°C with the drug. Then, the medium containing the adriamycin was removed, the cells were washed and fresh medium containing 80 *µ*g/ml of c-myb antisense (SEQ ID NO:5) or sense (SEQ ID NO:6) oligodeoxynucleotide was added. The antisense oligomer is complementary to an 18-nucleotide stretch of the c-myb mRNA transcript beginning with the translation initiation codon and extending downstream thereof. After 18 hours of incubation at 37°C, a second dose (40 *µ*g/ml) of oligodeoxynucleotides was added. After 9 days of incubation in the presence of the oligodeoxynucleotides, the cells were detached from the plate bottom by trypsinization and counted in trypan blue. The results, set forth in Fig. 10A (0.5 *µ*g/ml adriamycin) and 10B (1 *µ*g/ml adriamycin), represent the mean ± the standard deviation from two independent experiments.

### Example 13

### Effect of c-myb Antisense Oligonucleotide and 5-Fluorouracil on LOVO Cells

LOVO cells, which comprise a human colon carcinoma cell line, were seeded in a 24-well plate (Costar Corp., Cambridge, MA) in a concentration of 10⁴ cells/ml of F-12 medium plus 10% FBS. After overnight incubation, which allows the cells to attach to the bottom of the plate, 2.5 or 5 *µ*g/ml 5-fluorouracil (Roche Laboratories) were added and the cells incubated for 60 min. at 37°C with the drug. Then, the medium containing the adriamycin was removed, the cells were washed and fresh medium containing 80 *µ*g/ml of c-myb sense (SEQ ID NO:6) or antisense (SEQ ID NO:5) oligodeoxynucleotide was added. After 18 hours of incubation at 37°C, a second dose (40 *µ*g/ml) of oligodeoxynucleotides was added. After 9 days of incubation in the presence of the oligodeoxynucleotides, the cells were detached from the plate bottom by trypsinization and counted in trypan blue. The results, set forth in Fig. 11A (0.5 *µ*g/ml adriamycin) and 11B (1 *µ*g/ml adriamycin), represent the mean ± the standard deviation from two independent experiments.

### Example 14

### Bone Marrow Purging with Antisense Oligonucleotide/non-Antisense Drug Combination

Bone marrow is harvested from the iliac bones of a donor under general anesthesia in an operating room using standard techniques. Multiple aspirations are taken into heparinized syringes. Sufficient marrow is withdrawn so that the marrow recipient will be able to receive about 4 x 10⁸ to about 8 x 10⁸ processed marrow cells per kg of body weight. Thus, about 750 to 1000 ml of marrow is withdrawn. The aspirated marrow is transferred immediately into a transport medium (TC-199, Gibco, Grand Island, New York) containing 10,000 units of preservative-free heparin per 100 ml of medium. The aspirated marrow is filtered through three progressively finer meshes until a single cell suspension results, i.e., a suspension devoid of cellular aggregates, debris and bone particles. The filtered marrow is then processed further into an automated cell separator (e.g., Cobe 2991 Cell Processor) which prepares a "buffy coat" product, (i.e., leukocytes devoid of red cells and platelets). The buffy coat preparation is then placed in a transfer pack for further processing and storage. It may be stored until purging in liquid nitrogen using standard procedures. Alternatively, purging can be carried out immediately, then the purged marrow may be stored frozen in liquid nitrogen until it is ready for transplantation.

The purging procedure may be carried out as follows. Cells in the buffy coat preparation are adjusted to a cell concentration of about 2 x 10⁷/ml in TC-199 containing about 20% autologous plasma. Mafosfamide, for example, in a concentration of 25-50 *µ*g/ml, is added to the transfer packs containing the cell suspension. The cells are incubated for approximately 30 minutes at 37°C. The cells are then washed with physiologic buffer to remove the drug. Bcr-abl antisense oligodeoxynucleotide, for example, in a concentration of about 200 to 250 *µ*g/ml, is then added. The transfer packs are then placed in a 37°C waterbath and incubated for 18 - 24 hours with gentle shaking. The cells may then either be frozen in liquid nitrogen or washed once at 4°C in TC-199 containing about 20% autologous plasma to remove unincorporated oligomer. Washed cells are then infused into the recipient. Care must be taken to work under sterile conditions wherever possible and to maintain scrupulous aseptic techniques at all times.

### Example 15

### Distribution of BCR-ABL Antisense Oligonucleotides in Mouse Tissues

The following study demonstrates the in vivo stability of therapeutic antisense oligonucleotides.

### A. Phosphorothioate Antisense Oligodeoxynucleotide Tissue Distribution.

Mice were injected intravenously with b2a2 antisense oligodeoxynucleotides (1 mg/day, containing 1 *µ*g of uniformly ³⁵S-labelled oligodeoxynucleotides) for 9 consecutive days. Five mice were sacrificed, two at 24 hours, one at 72 hours, one at 7 days, and one at 14 days after the last injection. Single-cell suspensions from peripheral blood, bone marrow, spleen, kidney, liver and brain were prepared, washed twice with IMDM and used for DNA isolation to determine the presence of intact oligodeoxynucleotides. An equal amount of DNA (5 *µ*g) from each organ was electrophoresed in a 15% polyacrylamide-7M urea gel, electroblotted onto 0.1 *µ*m Nytran membranes (Schleicher and Schuell) and baked under vacuum at 80°C for 30 min. Filters were prehybridized and then hybridized with a ³²P-end-labeled oligodeoxynucleotide complementary to the injected b2a2 antisense oligodeoxynucleotide at 37°C in 5X SSC, 0.1% SDS and 100 *µ* g/ml salmon sperm DNA for 18 hours. Filters were then washed twice in 3X SSC and 0.1% SDS at 37°C and autoradiographed. The results are shown in Fig. 13A. The control lane contained about 10 ng of stock oligodeoxynucleotide used for the i.v. injection.

The bcr-abl oligodeoxynucleotides injected into mice were distributed, in intact form, throughout the body, but became concentrated in the liver. Lower levels were detected in kidney, spleen, lung, bone marrow and peripheral blood cells (Fig. 13A). Oligodeoxynucleotides were undetectable in the brain, in good agreement with a previous study of tissue distribution of phosphorothioate oligodeoxynucleotide complementary to the HIV tat splice acceptor site (Agrawal et al., Proc. Nat. Acad. Sci. USA 88, 7595-99, 1981). Intact oligodeoxynucleotides were detected in the kidney and in the liver up to 14 days after the last injection. (Fig. 13B).

Accumulation of bcr-abl phosphorothioate oligodeoxynucleotide in various organs was also assessed measuring the amount of ³⁵S-labeled material in weighed organ samples. To determine ³⁵S-concentrations, tissues were solubilized in Soluene-350 (Amersham 1 ml per 0.5 g), then mixed with 4.0 ml of scintillation cocktail (Hydrocount LSD, Baker) and radioactivity was measured in a Beckman LS7500 liquid scintillation counter. Oligodeoxynucleotide concentrations (³⁵S-*µ*M per gram of tissue) were derived dividing cpm/tissue by the specific activity of the injected oligodeoxynucleotide (cpm/*µ* mole). Since tissues have a density near 1 gram/ml, the data may be presented in molar terms (moles/liter). Tissues concentrations correlated with the relative levels of intact oligodeoxynucleotides detected in the same tissues and ranged from 3 to 26 *µ*M (Fig. 13A). Because phosphorothioate oligodeoxynucleotides undergo relatively slow degradation in mice tissues, the 9-day treatment schedule in SCID mice appeared to reach tissue concentrations, in every tissue except brain, that are highly effective in inhibiting BV173 cell proliferation and are adequate in inhibiting the growth of primary leukemic cells while sparing that of normal cells. Indeed, extracellular concentrations of bcr-abl antisense oligodeoxynucleotides in the 4-30 *µ*M range were not significantly toxic in vitro for colony-forming cells derived from bone marrow of healthy human donors, but efficiently inhibited (60-90% inhibition compared to sense oligodeoxynucleotide-treated samples) growth of primary leukemic cells from several CML-BC patients (Szcaylik et al., Science 253, 562-565, 1991).

### B. Cytoplasmic and Nuclear Uptake of Phosphorothioate Antisense Oligodeoxynucleotide

To obtain a more convincing demonstration that intact oligomers in tissues were in fact intracellular and not simply membrane-bound, we separated membrane, cytoplasmic and nuclear fractions from spleen, kidney and liver cell suspensions. Twenty-four hours after the last bcr-abl injection antisense oligodeoxynucleotide injection, one mouse was sacrificed and single-cell suspensions from spleen, kidney and liver were prepared, washed twice with IMDM and used (10⁶ cells/sample) to obtain membrane (C), cytoplasmic (B) and nuclear (A) fractions under fractionation conditions as described in Molecular Cloning, J. Sambrook et al. eds., 2nd ed. 1989, demonstrating about 90% cytoplasmic GAP protein in the cytoplasmic fraction (not shown). Nucleic acids extracted from each fraction were electrophoresed, electroblotted and hybridized as described above. The control lane contained ~ 1 ng of stock oligodeoxynucleotide used for the i.v. injection. The results shown in Fig. 13C indicate that most of the intact bcr-abl antisense oligodeoxynucleotide is detected in the nuclear and cytoplasmic fractions.

All references cited with respect to synthetic, preparative and analytical procedures are incorporated herein by reference.

The present invention may be embodied in other specific forms without departing from the spirit or essential attributes thereof and, accordingly, reference should be made to the appended claims, rather than to the foregoing specification, as indicating the scope of the invention.

## Claims

1. Pharmaceutical composition comprising:
(a) at least one oligonucleotide which has a nucleotide sequence complementary to at least a portion of the mRNA transcript of an oncogene or proto-oncogene, said oligonucleotide being hybridizable to said mRNA transcripts
(b) at least one antineoplastic chemotherapeutic agent other than an antisense oligonucleotide.

2. A composition according to claim 1 wherein the oligonucleotide is complementary to a portion of the mRNA of a gene selected from the group consisting of c-myc, L-myc, N-myc, cyclin D1, c-erbB, c-erbB2, c-fos, p53, c-myb, c-abl, c-kit, bcr-abl, N-ras, c-Ha-ras, K-ras, proliferating cellular nuclear antigen, and neu.

3. A composition according to claim 2 wherein the oligonucleotide is complementary to a portion of the mRNA of c-myb.

4. A composition according to claim 2 wherein the oligonucleotide is complementary to a portion of the mRNA of c-abl.

5. A composition according to claim 2 wherein the oligonucleotide is complementary to a portion of the mRNA of bcr-abl.

6. A composition according to claim 5 wherein the oligonucleotide is complementary to a portion of the bcr-abl mRNA corresponding to the breakpoint junction between the bcr-derived and abl-derived portions of said mRNA.

7. A composition according to claim 6 wherein the oligonucleotide is from a 13-mer to a 26-mer and the bcr-abl mRNA target sequence to which the antisense oligonucleotide hybridizes comprises from about 6 to about 13 abl-derived nucleotides, the balance of said target sequence comprising bcr-derived oligonucleotides.

8. A composition according to claim 1 wherein the antineoplastic chemotherapeutic agent is selected from the group consisting of antimetabolites, alkylating agents, plant alkaloids, antitumor antibiotics, and hormones.

9. A composition according to claim 1 wherein the antineoplastic chemotherapeutic agent is selected from the group consisting of methotrexate, trimetrexate, 5-fluorouracil, 5-fluorodeoxyuridine, cytosine arabinoside, 5-azacytidine, 6-mercaptopurine, 6-thioguanine, hydroxyurea, deoxycoformycin, nitrogen mustards, triethylenethiophosoporamide, busulfan, 4-hydroxyperoxycyclophosphoramide, mafosfamide, ifosfamide, melphalan, chlorambucil, cyclohexylnitrosourea, bis-chloroethylnitrosourea, methylcyclohexylnitrosourea, cis (II) platinum diamminedichloride, vinblastine, vincristine, vindesine, etoposide, teniposide, bleomycin, daunomycin, doxorubicin, epirubicin, idarubicin, esorubicin, mitomycin C, actinomycin D, mithramycin, prednisone, hydroxyprogesterone, testosterone, diethylstilbestrol, tamoxifen, dacarbazine, procarbazine, hexamethylmelamine, pentamethylmelamine, mitoxantrone, amsacrine, and combinations thereof.

10. A composition according to claim 9 wherein the antineoplastic chemotherapeutic agent is selected from the group consisting of methotrexate, 4-hydroxyperoxycyclophosphoramide, mafosfamide, cytosine arabinoside, bis-chloroethylnitrosourea, busulfan, etoposide, doxorubicin, cisplatin, and combinations thereof.

11. A composition according to claim 1 wherein the oligonucleotide is an at least 8-mer.

12. A composition according to claim 11 wherein the oligonucleotide is an alkylphosphonate oligonucleoside or phosphorothioate oligonucleotide.

13. A composition according to claim 11 wherein the oligonucleotide has a nucleotide sequence complementary to a portion of the mRNA lying within about 50 nucleotides of the translation initiation codon.

14. A composition according to claim 11 wherein the oligonucleotide comprises from a 12-mer to a 40-mer oligodeoxynucleotide.

15. A composition according to claim 14 wherein the oligodeoxynucleotide is an alkylphosphonate oligodeoxynucleoside or a phosphorothioate oligodeoxynucleotide.

16. A composition comprising:
(a) at least one oligonucleotide which has a nucleotide sequence complementary to at least a portion of the mRNA transcript of an oncogene or proto-oncogene, said oligonucleotide being hybridizable to said mRNA transcript; and
(b) at least one antineoplastic chemotherapeutic agent other than an antisense oligonucleotide;
for use in medicine.

17. A composition according to claim 16 wherein the oligonucleotide is as stated in any of claims 2 to 7 and 11 to 14.

18. A composition according to claim 16 wherein the oligonucleotide comprises from a 12-mer to a 40-mer oligodeoxynucleotide which is an alkylphosphonate oligodeoxynucleoside or a phosphorothioate oligodeoxynucleotide.

19. A composition according to claim 16 wherein the antineoplastic chemotherapeutic agent is as stated in any of claims 8 to 10.

20. Use of a composition comprising
(a) at least one oligonucleotide which has a nucleotide sequence complementary to at least a portion of the mRNA transcript of an oncogene or proto-oncogene, said oligonucleotide being hybridizable to said mRNA transcript; and
(b) at least one antineoplastic chemotherapeutic agent other than an antisense oligonucleotide;
in the manufacture of a medicament for use in the treatment of cancer.

21. Use of an oligonucleotide which has a nucleotide sequence complementary to at least a portion of the mRNA transcript of an oncogene or proto-oncogene, said oligonucleotide being hybridizable to said mRNA transcript, in the manufacture of a medicament for use, simultaneously or sequentially, with at least one antineoplastic chemotherapeutic agent other than an antisense oligonucleotide, in the treatment of cancer.

22. Use of an antineoplastic chemotherapeutic agent other than an antisense oligonucleotide in the manufacture of a medicament, for use simultaneously or sequentially, with at least one oligonucleotide which has a nucleotide sequence complementary to at least a portion of the mRNA transcript of an oncogene or proto-oncogene, said oligonucleotide being hybridizable to said mRNA transcript, in the treatment of cancer.

23. Use according to any of claims 20 to 22 wherein the treatment is the purging of bone marrow of neoplastic cells.

24. Use according to claim 20 or 21 wherein the oligonucleotide is as stated in any of claims 2 to 7 and 11 to 14.

25. Use according to claim 24 wherein the oligonucleotide comprises from a 12-mer to a 40-mer oligodeoxynucleotide which is an alkylphosphonate oligodeoxynucleoside or a phosphorothioate oligodeoxynucleotide.

26. Use according to claims 20 or 22 wherein the antineoplastic chemotherapeutic agent is as stated in any of claims 8 to 10.

27. A method for purging bone marrow of neoplastic cells comprising:
treating bone marrow cells aspirated from an individual afflicted with a neoplastic disease with an effective amount of
(a) at least one oligonucleotide which has a nucleotide sequence complementary to at least a portion of the mRNA transcript of an oncogene or proto-oncogene, said oligonucleotide being hybridizable to said mRNA transcript; and
(b) at least one antineoplastic chemotherapeutic agent other than an antisense oligonucleotide.

28. An in vitro method for inhibiting the proliferation of neoplastic cells charaterized by the amplification or expression of a target oncogene or proto-oncogene comprising introducing into such cells an artificially-constructed gene which, upon transcription in said cells, produces RNA complementary to the mRNA transcript of the target oncogene or proto-oncogene, and treating such cells with at least one chemotherapeutic agent other than an antisense oligonucleotide.

29. A method according to claim 28 wherein the artificially-constructed gene is introduced into said cells by transfection, by a transducing viral vector or by microinjection.

## Patentansprüche

1. Pharmazeutische Zusammensetzung enthaltend:
(a) wenigstens ein Oligonukleotid, welches eine Nukleotidsequenz aufweist, die komplementär zu wenigstens einem Bereich des mRNA-Transkripts eines Onkogens oder Proto-Onkogens ist, wobei das Oligonukleotid zu dem mRNA-Transkript hybridisierbar ist;
(b) wenigstens ein antineoplastisches chemotherapeutisches Mittel mit Ausnahme eines Antisense-Oligonukleotids.

2. Zusammensetzung nach Anspruch 1, **dadurch gekennzeichnet**, dass das Oligonukleotid komplementär zu einem Bereich der mRNA eines Gens ausgewählt aus der Gruppe bestehend aus c-myc, L-myc, N-myc, Cyclin D1, c-erbB, c-erbB2, c-fos, p53, c-myb, c-abl, c-kit, bcr-abl, N-ras, c-Ha-ras, K-ras, proliferierendes zelluläres Kern-Antigen und neu ist.

3. Zusammensetzung nach Anspruch 2, **dadurch gekennzeichnet**, dass das Oligonukleotid komplementär zu einem Bereich der mRNA von c-myb ist.

4. Zusammensetzung nach Anspruch 2, **dadurch gekennzeichnet,** dass das Oligonukleotid komplementär zu einem Bereich der mRNA von c-abl ist.

5. Zusammensetzung nach Anspruch 2, **dadurch gekennzeichnet,** dass das Oligonukleotid komplementär zu einem Bereich des mRNA von bcr-abl ist.

6. Zusammensetzung nach Anspruch 5, **dadurch gekennzeichnet,** dass das Oligonukleotid komplementär zu einem Bereich der bcr-abl mRNA ist, die mit der Bruchstellenverbindung zwischen den bcr-abgeleiteten und abl-abgeleiteten Bereichen der mRNA korrespondiert.

7. Zusammensetzung nach Anspruch 6, **dadurch gekennzeichnet**, dass das Oligonukleotid eine 13-mer bis ein 26-mer ist und die bcr-abl mRNA Zielsequenz, zu welcher das Antisense-Oligonukleotid hybridisiert, von ungefähr 6 bis ungefähr 13 abl-abgeleitete Nukleotide aufweist, wobei der Rest der Zielsequenz bcr-abgeleitete Oligonukleotide aufweist.

8. Zusammensetzung nach Anspruch 1, **dadurch gekennzeichnet,** dass das antineoplastische chemotherapeutische Mittel ausgewählt ist aus der Gruppe bestehend aus Antimetaboliten, Alkylierungsmitteln, Pflanzenalkaloiden, Antitumor-Antibiotika und Hormonen.

9. Zusammensetzung nach Anspruch 1, **dadurch gekennzeichnet,** dass das antineoplastische chemotherapeutische Mittel ausgewählt ist aus der Gruppe bestehend aus Methotrexat, Trimetrexat, 5-Fluorouracil, 5-Fluorodeoxyuridin, Cytosinarabinosid, 5-Azacytidin, 6-Mercaptopurin, 6-Thioguanin, Hydroxyharnstoff, Deoxycoformycin, Stickstoffsenfe, Triethylenthiophosphoramid, Busulfan, 4-Hydroxyperoxycyclophosphoramid, Mafosfamid, Ifosfamid, Melphalan, Chlorambucil, Cyclohexylnitrosoharnstoff, Bis-chloroethylnitrosoharnstoff, Methylcyclohexylnitrosoharnstoff, cis (II) Platindiammindichlorid, Vinblastin, Vincristin, Vindesin, Etoposid, Teniposid, Bleomycin, Daunomycin, Doxorubicin, Epirubincin, Idarubicin, Esorubicin, Mitomycin C, Actinomycin D, Mithramycin, Prednison, Hydroxyprogesterone, Testosteron, Diethylstilbestrol, Tamoxifen, Dacarbazin, Procarbazin, Hexamethylmelamin, Pentamethylmelamin, Mitoxantron, Amsacrin und Kombinationen von diesen.

10. Zusammensetzung nach Anspruch 9, **dadurch gekennzeichnet**, dass das antineoplastische chemotherapeutische Mittel ausgewählt ist aus der Gruppe bestehend aus Methotrexat, 4-Hydroxyperoxycyclophosphoramide, Mafosfamid, Cytosinarabinosid, Bischloroethylnitrosoharnstoff, Busulfan, Etoposid, Doxorubicin, cis-Platin und Kombinationen derselben.

11. Zusammensetzung nach Anspruch 1, **dadurch gekennzeichnet**, dass das Oligonukleotid wenigstens ein 8-mer ist.

12. Zusammensetzung nach Anspruch 11, **dadurch gekennzeichnet**, dass das Oligonukleotid ein Alkylphosphonat-Oligonukleosid oder Phosphorotioat-Oligonukleotid ist.

13. Zusammensetzung nach Anspruch 11, **dadurch gekennzeichnet**, dass das Oligonukleotid eine Nukleotidsequenz aufweist, die komplementär zu einem Bereich der mRNA ist, die innerhalb ungefähr 50 Nukleotide des Translations-Initiationscodon liegt.

14. Zusammensetzung nach Anspruch 11, **dadurch gekennzeichnet**, dass das Oligonukleotid ein 12-mer bis 40-mer Oligodeoxynukleotid aufweist.

15. Zusammensetzung nach Anspruch 14, **dadurch gekennzeichnet**, dass das Oligodeoxynukleotid ein Alkylphosphonat-Oligodeoxynukleosid oder ein Phosphorothioat-Oligodeoxynukleotid ist.

16. Zusammensetzung enthaltend:
(a) wenigstens ein Oligonukleotid, welches eine Nukleotidsequenz aufweist, die komplementär zu wenigstens einem Bereich des mRNA-Transkripts eines Onkogens oder Proto-Onkogens ist, wobei das Oligonukleotid zu dem mRNa-Transkript hybridisierbar ist; und
(b) wenigstens ein antineoplastisches chemotherapeutisches Mittel mit Ausnahme eines Antisense-Oligonukleotides;
zur medizinischen Verwendung.

17. Zusammensetzung nach Anspruch 16, **dadurch gekennzeichnet**, dass das Oligonukleotid ein solches wie in einem der Ansprüche 2 bis 7 und 11 bis 14 ist.

18. Zusammensetzung nach Anspruch 16, **dadurch gekennzeichnet**, dass das Oligonukleotid ein 12-mer bis zu einem 40-mer Oligodeoxynukleotid aufweist, welches ein Alkylphosphonat-Oligodeoxynukleosid oder ein Phosphorothioat-Oligodeoxynukleotid ist.

19. Zusammensetzung nach Anspruch 16, **dadurch gekennzeichnet**, dass das antineoplastische chemotherapeutische Mittel ein solches nach einem der Ansprüche 8 bis 10 ist.

20. Verwendung einer Zusammensetzung enthaltend
(a) wenigstens ein Oligonukleotid, welches eine Nukleotidsequenz aufweist, die komplementär zu wenigstens einem Bereich des mRNA-Transkripts eines Onkogens oder Proto-Onkogens ist, wobei das Oligonukleotid zu dem mRNA-Transkript hybridisierbar ist; und
(b) wenigstens ein antineoplastisches chemotherapeutisches Mittel mit Ausnahme von einem Antisense-Oligonukleotid;
bei der Herstellung eines Arzneimittels zur Verwendung bei der Behandlung von Krebs.

21. Verwendung eines Oligonukleotides, welches eine Nukleotidsequenz aufweist, die komplementär zu wenigstens einem Bereich des mRNA-Transkripts eines Onkogens oder Proto-Onkongens ist, wobei das Oligonukleotid zu dem mRNA-Transkript hybridisierbar ist, bei der Herstellung eines Arzneimittels zur gleichzeitigen oder aufeinanderfolgenden Verwendung mit wenigstens einem antineoplastischen chemotherapeutischen Mittel mit Ausnahme eines Antisense-Oligonukleotides bei der Behandlung von Krebs.

22. Verwendung eines antineoplastischen chemotherapeutischen Mittels mit Unterschied eines Antisense-Oligonukleotides bei der Herstellung eines Arzneimittels zur gleichzeitigen oder nacheinander erfolgenden Verwendung mit wenigstens einem Oligonukleotid, welches eine Nukleotidsequenz aufweist, die komplementär zu wenigstens einem Bereich des mRNA-Transkripts eines Onkogens oder Proto-Onkogens ist, wobei das Oligonukleotid zu dem mRNA-Transkript hybridisierbar ist, bei der Behandlung von Krebs.

23. Verwendung nach einem der Ansprüche 20 bis 22, wobei die Behandlung die Reinigung des Knochenmarks von neoplastischen Zellen darstellt.

24. Verwendung nach Anspruch 20 oder 21, **dadurch gekennzeichnet**, dass das Oligonukleotid eines der in den Ansprüchen 2 bis 7 und 11 bis 14 genannten ist.

25. Verwendung nach Anspruch 24, **dadurch gekennzeichnet**, dass das Oligonukleotid ein 12-mer bis zu einem 40-mer Oligodeoxynukleotid aufweist, welches ein Alkylphosphonat-Oligodeoxynukleosid oder ein Phosphorothioat-Oligodeoxynukleotid ist.

26. Verwendung nach Anspruch 20 oder 22, **dadurch gekennzeichnet**, dass das antineoplastische chemo-therapeutische Mittel eines der in den Ansprüchen 8 bis 10 genannten ist.

27. Verfahren zur Reinigung des Knochenmarks von neoplastischen Zellen, umfassend:
die Behandlung von Knochenmarkszellen, die von einem mit einer neoplastischen Krankheit befallenen Individuum aspiriert wurden, mit einer wirksamen Menge von
(a) wenigstens einem Oligonukleotid, welches eine Nukleotidsequenz aufweist, die komplementär zu wenigstens einem Bereich des mRNA-Transkripts eines Onkogens oder Proto-Onkogens ist, wobei das Oligonukleotid zu dem mRNA-Transkript hybridisierbar ist; und
(b) wenigstens einem antineoplastischen chemotherapeutischen Mittel mit Ausnahme von einem Antisense-Oligonukleotid.

28. Ein in vitro-Verfahren zur Inhibierung der Proliferation von neoplastischen Zellen, gekennzeichnet durch die Amplifikation oder Expression eines Zielonkogens oder Proto-Onkogens, umfassend die Einführung eines artifiziell konstruierten Gens in derartige Zellen, welches bei der Transkription in diesen Zellen RNA produziert, die komplementär zu dem mRNA-Transkript des Zielonkogens oder Proto-Onkogens ist, und Behandlung derartiger Zellen mit wenigstens einem chemotherapeutischen Mittel ausgenommen eines Antisense-Oligonukleotids.

29. Verfahren nach Anspruch 28, **dadurch gekennzeichnet**, dass das artifiziell konstruierte Gen in die Zellen durch Transfektion mittels eines transduzierenden viralen Vektors oder durch Mikroinjektion eingeführt wird.

## Revendications

1. Composition pharmaceutique comprenant :
(a) au moins un oligonucléotide qui a une séquence nucléotidique complémentaire d'au moins une partie du transcrit ARNm d'un oncogène ou proto-oncogène, cet oligonucléotide pouvant s'hybrider au transcrit ARNm ;
(b) au moins un agent chimiothérapeutique anti-néoplasique autre qu'un oligonucléotide antisens.

2. Composition selon la revendication 1,
caractérisée en ce que
l'oligonucléotide est complémentaire d'une partie de l'ARNm d'un gène choisi parmi le groupe constitué de c-myc, L-myc, N-myc, cycline D1, C-erbB, c-erbB2, c-fos, p53, c-myb, c-abl, c-kit, bcr-abl, N-ras, c-Ha-ras, K-ras, l'antigène nucléaire de prolifération cellulaire, et neu.

3. Composition selon la revendication 2,
caractérisée en ce que
l'oligonucléotide est complémentaire d'une partie de l'ARNm de c-myb.

4. Composition selon la revendication 2,
caractérisée en ce que
l'oligonucléotide est complémentaire d'une partie de l'ARNm de c-abl.

5. Composition selon la revendication 2,
caractérisée en ce que
l'oligonucléotide est complémentaire d'une partie de l'ARNm de bcr-abl.

6. Composition selon la revendication 5,
caractérisée en ce que
l'oligonucléotide est complémentaire d'une partie de l'ARNm bcr-abl correspondant à la jonction du point de rupture entre les parties provenant du gène bcr et provenant du gène abl de l'ARNm.

7. Composition selon la revendication 6,
caractérisée en ce que
l'oligonucléotide a une longueur de 13-mer à 26-mer et la séquence cible de l'ARNm bcr-abl à laquelle l'oligonucléotide s'hybride comprend d'environ 6 à environ 13 nucléotides provenant du gène abl, l'équilibre de cette séquence cible comprenant des oligonucléotides provenant du gène bcr.

8. Composition selon la revendication 1,
caractérisée en ce que
l'agent chimiothérapeutique anti-néoplasique est choisi parmi le groupe constitué d'antimétabolites, d'agents alkylant, d'alcaloïdes de plantes, d'antibiotiques antitumoraux, et d'hormones.

9. Composition selon la revendication 1,
caractérisée en ce que
l'agent chimiothérapeutique anti-néoplasique est choisi parmi le groupe constitué de méthotrexate, trimétrexate, 5-fluorouracile, 5-fluorodésoxyuridine, cytosine arabinoside, 5-azacytidine, 6-mercaptopurine, 6-thioguanine, hydroxyurée, désoxycoformycine, moutarde azotée, triéthylènethiophosphoramide, busulfane, 4-hydroxypéroxycyclophosphoramide, mafosfamide, ifosfamide, melphalane, chlorambucile, cyclohexylnitrourée, bis-chloroéthylnitrourée, méthylcyclohexylnitrourée, cis-(II)-platinium diaminedichloride, vinblastine, vincristine, vindésine, étoposide, téniposide, bléomycine, daunomycine, doxorubicine, épirubicine, idarubicine, ésorubicine, mitomycine C, actinomycine D, mithramicyne, prednisone, hydroxyprogestérone, testostérone, dihéthylstilbestrol, tamoxifène, dacarbazine, procarbazine, hexaméthylmélamine, pentaméthylmélamine, mitoxantrone, amsacrine, et leurs combinaisons.

10. Composition selon la revendication 9,
caractérisée en ce que
l'agent chimiothérapeutique antinéoplasique est choisi parmi le groupe constitué du méthotrexate, 4-hydroxypéroxycyclophosphoramide, mafosfamide, cytosine arabinoside, bis-chloroéthylnitrourée, busulfane, étoposide, doxorubicine, cisplastine, et leurs combinaisons.

11. Composition selon la revendication 1,
caractérisée en ce que
l'oligonucléotide a une longueur d'au moins 8-mer.

12. Composition selon la revendication 11,
caractérisée en ce que
l'oligonucléotide est un oligonucléotide de type alkylphosphonate ou un oligonucléotide de type phosphorothioate.

13. Composition selon la revendication 11,
caractérisée en ce que
l'oligonucléotide a une séquence nucléotidique complémentaire d'une partie de l'ARNm se trouvant à l'intérieur d'environ 50 nucléotides du codon d'initiation de traduction.

14. Composition selon la revendication 11,
caractérisée en ce que
l'oligonucléotide comprend un oligodésoxynucléotide de 12-mer à 40-mer.

15. Composition selon la revendication 14,
caractérisée en ce que
l'oligodésoxynucléotide est un oligodésoxynucléotide de type alkylphosphonate ou un oligodésoxynucléotide de type phosphorothioate.

16. Composition comprenant :
(a) au moins un oligonucléotide qui a une séquence nucléotidique complémentaire d'au moins une partie du transcrit ARNm d'un oncogène ou proto-oncogène, cet oligonucléotide pouvant s'hybrider au transcrit ARNm ;
(b) au moins un agent chimiothérapeutique anti-néoplasique autre qu'un oligonucléotide antisens,
pour utilisation en médecine.

17. Composition selon la revendication 16,
caractérisée en ce que
l'oligonucléotide est tel que décrit selon l'une quelconque des revendications 2 à 7 et 11 à 14.

18. Composition selon la revendication 16,
caractérisée en ce que
l'oligonucléotide comprend un oligonucléotide de 12-mer à 40-mer qui est un oligodésoxynucléotide de type alkylphosphonate ou un oligodésoxynucléotide de type phosphorothioate.

19. Composition selon la revendication 16,
caractérisée en ce que
l'agent chimiothérapeutique anti-néoplasique est tel que décrit selon l'une quelconque des revendications 8 à 10.

20. Utilisation d'une composition comprenant :
(a) au moins un oligonucléotide qui a une séquence nucléotidique complémentaire d'au moins une partie du transcrit ARNm d'un oncogène ou proto-oncogène, cet oligocnucléotide pouvant s'hybrider au transcrit ARNm ;
(b) au moins un agent chimiothérapeutique anti-néoplasique autre qu'un oligonucléotide antisens ;
pour la fabrication d'un médicament utile dans le traitement du cancer.

21. Utilisation d'un oligonucléotide qui a une séquence nucléotidique complémentaire d'au moins une partie du transcrit ARNm d'un oncogène ou proto-oncogène, cet oligonucléotide pouvant s'hybrider au transcrit ARNm , pour la fabrication d'un médicament utile, simultanément ou séquentiellement, avec un agent chimiothérapeutique antinéoplasique autre qu'un oligonucléotide antisens, dans le traitement du cancer.

22. Utilisation d'un agent chimiothérapeutique antinéoplasique autre qu'un oligonucléotide antisens pour la fabrication d'un médicament, utilisé simultanément ou séquentiellement, avec au moins un oligonucléotide qui a une séquence nucléotidique complémentaire d'au moins une partie du transcrit ARNm d'un oncogène ou proto-ancogène, cet oligonucléotide pouvant s'hybrider au transcrit ARNm, dans le traitement du cancer.

23. Utilisation selon l'une selon l'une quelconque des revendications 20 à 22,
caractérisée en ce que
le traitement est une purification de la moelle osseuse des cellules néoplasiques.

24. Utilisation selon l'une quelconque des revendications 20 ou 21,
caractérisée en ce que
l'oligonucléotide est tel que décrit selon l'une quelconque des revendications 2 à 7 et 11 à 14.

25. Utilisation selon la revendication 24,
caractérisée en ce que
l'oligonucléotide comprend un oligonucléotide de 12-mer à 40-mer qui est un oligodésoxynucléotide de type alkylphosphonate ou un oligodésoxynucléotide de type phosphorothioate.

26. Utilisation selon l'une quelconque des revendications 20 ou 22,
caractérisée en ce que
l'agent chimiothérapeutique anti-néoplasique est tel que décrit selon l'une quelconque des revendications 8 à 10.

27. Méthode pour purifier la moelle osseuse des cellules néoplasiques comprenant :
le traitement des cellules de la moelle osseuse aspirée d'un individu malade d'une maladie néoplasique, avec une quantité efficace de
(a) au moins un oligonucléotide qui a une séquence nucléotidique complémentaire d'au moins une partie du transcrit ARNm d'un oncogène ou proto-oncogène, cet oligonucléotide pouvant s'hybrider au transcrit ARNm ;
(b) au moins un agent chimiothérapeutique anti-néoplasique autre qu'un oligonucléotide antisens.

28. Méthode in vitro pour inhiber la prolifération de cellules néoplasiques caractérisée par l'amplification ou l'expression d'un oncogène ou proto-oncogène cible, comprenant :
- l'introduction dans ces cellules d'un gène construit artificiellement qui, au cours de la transcription dans les cellules, produit un ARN complémentaire du transcrit ARNm de l'oncogène ou proto-oncogène cible, et
- le traitement des cellules avec au moins un agent chimiothérapeutique autre qu'un oligonucléotide antisens.

29. Méthode selon la revendication 28,
caractérisée en ce que
le gène construit artificiellement est introduit à l'intérieur des cellules par transfection, par un vecteur viral de transduction ou par micro-injection.


### SEQUENCE LISTING

(1) GENERAL INFORMATION:
   (i) APPLICANT:
      (A) NAME: Temple University - Of the Commonwealth System of Higher Education
      (B) STREET: Broad Street and Montgomery Avenue
      (C) CITY: Philadelphia
      (D) STATE: Pennsylvania
      (E) COUNTRY: United States of America
      (F) POSTAL CODE (ZIP): PA 19122
   (i) APPLICANT:
      (A) NAME: Thomas Jefferson University
      (B) STREET: 1020 Locust Street
      (C) CITY: Philadelphia
      (D) STATE: Pennsylvania
      (E) COUNTRY: United States of America
      (F) POSTAL CODE (ZIP): PA 19107
   (ii) TITLE OF INVENTION: Combination of Antineoplastic
      Agent and Antisense Oligonucleotides for Treatment of Cancer
   (iii) NUMBER OF SEQUENCES: 25
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
   (v) CURRENT APPLICATION DATA:
      APPLICATION NUMBER: EP 93919965.9
   (vi) PRIOR APPLICATION DATA:
      (A) APPLICATION NUMBER: US 965671
      (B) FILING DATE: 21-OCT-1992
   (vi) PRIOR APPLICATION DATA:
      (A) APPLICATION NUMBER: WO PCT/US93/07541
      (B) FILING DATE: 10-AUG-1993
(2) INFORMATION FOR SEQ ID NO:1:
   (i) SEQUENCE CHARACTERISTICS:
      (A) LENGTH: 18 Nucleotides
      (B) TYPE: nucleic acid
      (C) STRANDEDNESS: single stranded
      (D). TOPOLOGY: linear
   (xi) SEQUENCE DESCRIPTION: SEQ ID NO:1:
   GAAGGGCTTC TTCCTTAT 18
(2) INFORMATION FOR SEQ ID NO:2:
   (i) SEQUENCE CHARACTERISTICS:
      (A) LENGTH: 18 Nucleotides
      (B) TYPE: nucleic acid
      (C) STRANDEDNESS: single stranded
      (D) TOPOLOGY: linear
   (xi) SEQUENCE DESCRIPTION: SEQ ID NO:2:
   ATAAGGAAGA AGCCCTTC 18
(2) INFORMATION FOR SEQ ID NO:3:
   (i) SEQUENCE CHARACTERISTICS:
      (A) LENGTH: 18 Nucleotides
      (B) TYPE: nucleic acid
      (C) STRANDEDNESS: single stranded
      (D) TOPOLOGY: linear
   (xi) SEQUENCE DESCRIPTION: SEO ID NO:3:
   GAAGGGCTTT TGAACTCT 18
(2) INFORMATION FOR SEQ ID NO:4:
   (i) SEQUENCE CHARACTERISTICS:
      (A) LENGTH: 18 Nucleotides
      (B) TYPE: nucleic acid
      (C) STRANDEDNESS: single stranded
      (D) TOPOLOGY: linear
   (xi) SEQUENCE DESCRIPTION: SEQ ID NO:4:
   AGAGTTCAAA AGCCCTTC 18
(2) INFORMATION FOR SEQ ID NO:5:
   (i) SEQUENCE CHARACTERISTICS:
      (A) LENGTH: 18 Nucleotides
      (B) TYPE: nucleic acid
      (C) STRANDEDNESS: single stranded
      (D) TOPOLOGY: linear
   (xi) SEQUENCE DESCRIPTION: SEQ ID NO:5:
   GTGCCGGGGT CTTCGGGC 18
(2) INFORMATION FOR SEQ ID NO:6:
   (i) SEQUENCE CHARACTERISTICS:
      (A) LENGTH: 18 Nucleotides
      (B) TYPE: nucleic acid
      (C) STRANDEDNESS: double stranded
      (D) TOPOLOGY: linear
   (xi) SEQUENCE DESCRIPTION: SEQ ID NO:6:
   GCCCGAAGAC CCCGGCAC 18
(2) INFORMATION FOR SEQ ID NO:7
   (i) SEQUENCE CHARACTERISTICS:
      (A) LENGTH: 14 Nucleotides
      (B) TYPE: nucleic acid
      (C) STRANDEDNESS: single stranded
      (D) TOPOLOGY: linear
   (xi) SEQUENCE DESCRIPTION: SEO ID NO:7:
   AGGGCTTCTT CCTT 14
(2) INFORMATION FOR SEQ ID NO:8:
   (i) SEQUENCE CHARACTERISTICS:
      (A) LENGTH: 16 Nucleotides
      (B) TYPE: nucleic acid
      (C) STRANDEDNESS: single stranded
      (D) TOPOLOGY: linear
   (xi) SEQUENCE DESCRIPTION: SEQ ID NO:8:
   AAGGGCTTCT TCCTTA 16
(2) INFORMATION FOR SEQ ID NO:9:
   (i) SEQUENCE CHARACTERISTICS:
      (A) LENGTH: 20 Nucleotides
      (B) TYPE: nucleic acid
      (C) STRANDEDNESS: single stranded
      (D) TOPOLOGY: linear
   (xi) SEQUENCE DESCRIPTION: SEQ ID NO:9:
   TGAAGGGCTT CTTCCTTATT 20
(2) INFORMATION FOR SEQ ID NO:10:
   (i) SEQUENCE CHARACTERISTICS:
      (A) LENGTH: 22 Nucleotides
      (B) TYPE: nucleic acid
      (C) STRANDEDNESS: single stranded
      (D) TOPOLOGY: linear
   (xi) SEQUENCE DESCRIPTION: SEQ ID NO:10:
   CTGAAGGGCT TCTTCCTTAT TG 22
(2) INFORMATION FOR SEQ ID NO:11:
   (i) SEQUENCE CHARACTERISTICS:
      (A) LENGTH: 24 Nucleotides
      (B) TYPE: nucleic acid
      (C) STRANDEDNESS: single stranded
      (D) TOPOLOGY: linear
   (xi) SEQUENCE DESCRIPTION: SEQ ID NO:11:
   GCTGAAGGGC TTCTTCCTTA TTGA 24
(2) INFORMATION FOR SEQ ID NO:12:
   (i) SEQUENCE CHARACTERISTICS:
      (A) LENGTH: 26 Nucleotides
      (B) TYPE: nucleic acid
      (C) STRANDEDNESS: single stranded
      (D) TOPOLOGY: linear
   (xi) SEQUENCE DESCRIPTION: SEQ ID NO:12:
   CGCTGAAGGG CTTCTTCCTT ATTGAT 26
(2) INFORMATION FOR SEQ ID NO:13:
   (i) SEQUENCE CHARACTERISTICS:
      (A) LENGTH: 14 Nucleotides
      (B) TYPE: nucleic acid
      (C) STRANDEDNESS: single stranded
      (D) TOPOLOGY: linear
   (xi) SEQUENCE DESCRIPTION: SEQ ID NO:13:
   AGGGCTTTTG AACT 14
(2) INFORMATION FOR SEQ ID NO:14:
   (i) SEQUENCE CHARACTERISTICS:
      (A) LENGTH: 16 Nucleotides
      (B) TYPE: nucleic acid
      (C) STRANDEDNESS: single stranded
      (D) TOPOLOGY: linear
   (xi) SEQUENCE DESCRIPTION: SEQ ID NO:14:
   AAGGGCTTTT GAACTC 16
(2) INFORMATION FOR SEQ ID NO:15:
   (i) SEQUENCE CHARACTERISTICS:
      (A) LENGTH: 20 Nucleotides
      (B) TYPE: nucleic acid
      (C) STRANDEDNESS: single stranded
      (D) TOPOLOGY: linear
   (xi) SEQUENCE DESCRIPTION: SEO ID NO:15:
   TGAAGGGCTT TTGAACTCTG 20
(2) INFORMATION FOR SEQ ID NO:16:
   (i) SEQUENCE CHARACTERISTICS:
      (A) LENGTH: 22 Nucleotides
      (B) TYPE: nucleic acid
      (C) STRANDEDNESS: single stranded
      (D) TOPOLOGY: linear
   (xi) SEQUENCE DESCRIPTION: SEQ ID NO:16:
   CTGAAGGGCT TTTGAACTCT GC 22
(2) INFORMATION FOR SEQ ID NO:17:
   (i) SEQUENCE CHARACTERISTICS:
      (A) LENGTH: 24 Nucleotides
      (B) TYPE: nucleic acid
      (C) STRANDEDNESS: single stranded
      (D) TOPOLOGY: linear
   (xi) SEQUENCE DESCRIPTION: SEQ ID NO:17:
   GCTGAAGGGC TTTTGAACTC TGCT 24
(2) INFORMATION FOR SEQ ID NO:18:
   (i) SEQUENCE CHARACTERISTICS:
      (A) LENGTH: 26 Nucleotides
      (B) TYPE: nucleic acid
      (C) STRANDEDNESS: single stranded
      (D) TOPOLOGY: linear
   (xi) SEQUENCE DESCRIPTION: SEQ ID NO:18:
   CGCTGAAGGG CTTTTGAACT CTGCTT 26
(2) INFORMATION FOR SEQ ID NO:19:
   (i) SEQUENCE CHARACTERISTICS:
      (A) LENGTH: 14 Nucleotides
      (B) TYPE: nucleic acid
      (C) STRANDEDNESS: single stranded
      (D) TOPOLOGY: linear
   (xi) SEQUENCE DESCRIPTION: SEQ ID NO:19:
   AGGGCTTCTG CGTC 14
(2) INFORMATION FOR SEQ ID NO:20:
   (i) SEQUENCE CHARACTERISTICS:
      (A) LENGTH: 16 Nucleotides
      (B) TYPE: nucleic acid
      (C). STRANDEDNESS: single stranded
      (D) TOPOLOGY: linear
   (xi) SEQUENCE DESCRIPTION: SEQ ID NO:20:
   AAGGGCTTCT GCGTCT 16
(2) INFORMATION FOR SEQ ID NO:21:
   (i) SEQUENCE CHARACTERISTICS:
      (A) LENGTH: 18 Nucleotides
      (B) TYPE: nucleic acid
      (C) STRANDEDNESS: single stranded
      (D) TOPOLOGY: linear
   (xi) SEQUENCE DESCRIPTION: SEQ ID NO:21:
   GAAGGGCTTC TGCGTCTC 18
(2) INFORMATION FOR SEQ ID NO:22:
   (i) SEQUENCE CHARACTERISTICS:
      (A) LENGTH: 20 Nucleotides
      (B) TYPE: nucleic acid
      (C) STRANDEDNESS: single stranded
      (D) TOPOLOGY: linear
   (xi) SEQUENCE DESCRIPTION: SEQ ID NO:22:
   TGAAGGGCTT CTGCGTCTCC 20
(2) INFORMATION FOR SEQ ID NO:23:
   (i) SEQUENCE CHARACTERISTICS:
      (A) LENGTH: 22 Nucleotides
      (B) TYPE: nucleic acid
      (C) STRANDEDNESS: single stranded
      (D) TOPOLOGY: linear
   (xi) SEQUENCE DESCRIPTION: SEQ ID NO:23:
   CTGAAGGGCT TCTGCGTCTC CA 22
(2) INFORMATION FOR SEQ ID NO:24:
   (i) SEQUENCE CHARACTERISTICS:
      (A) LENGTH: 24 Nucleotides
      (B) TYPE: nucleic acid
      (C) STRANDEDNESS: single stranded
      (D) TOPOLOGY: linear
   (xi) SEQUENCE DESCRIPTION: SEQ ID NO:24:
   GCTGAAGGGC TTCTGCGTCT CCAT 24
(2) INFORMATION FOR SEQ ID NO:25:
   (i) SEQUENCE CHARACTERISTICS:
      (A) LENGTH: 26 Nucleotides
      (B) TYPE: nucleic acid
      (C) STRANDEDNESS: single stranded
      (D) TOPOLOGY: linear
   (xi) SEQUENCE DESCRIPTION: SEQ ID NO:25:
   CGCTGAAGGG CTTCTGCGTC TCCATG 26